(19) **Europäisches Patentamt**
**European Patent Office**
**Office européen des brevets**

(11) **EP 3 781 234 B1**

(12) **EUROPEAN PATENT SPECIFICATION**

(45) Date of publication and mention
of the grant of the patent:
**10.07.2024 Bulletin 2024/28**

(51) International Patent Classification (IPC):
**A61M 5/168** (2006.01)     **A61M 5/142** (2006.01)
**A61M 39/22** (2006.01)     **A61M 5/14** (2006.01)

(21) Application number: **19721950.4**

(22) Date of filing: **18.04.2019**

(52) Cooperative Patent Classification (CPC):
**A61M 5/16822; A61M 5/14216;** A61M 39/223;
A61M 2005/1403; A61M 2005/14208;
A61M 2039/229; A61M 2205/3379;
A61M 2205/3561; A61M 2205/3576; A61M 2205/50;
A61M 2205/505

(86) International application number:
**PCT/US2019/028139**

(87) International publication number:
**WO 2019/204617 (24.10.2019 Gazette 2019/43)**

(54) **METHOD OF REMOVAL OF GAS FROM RESERVOIR**

VERFAHREN ZUR ENTFERNUNG VON GAS AUS EINEM RESERVOIR

PROCÉDÉ D'ÉLIMINATION DE GAZ D'UN RÉSERVOIR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.04.2018 US 201862659984 P
28.08.2018 US 201862723792 P**

(43) Date of publication of application:
**24.02.2021 Bulletin 2021/08**

(73) Proprietor: **Bayer HealthCare LLC
Whippany, NJ 07981 (US)**

(72) Inventors:
• **MCDERMOTT, Michael
Pittsburgh, Pennsylvania 15237 (US)**
• **BARONE, William
Pittsburgh, Pennsylvania 15217 (US)**

(74) Representative: **BIP Patents
c/o Bayer Intellectual Property GmbH
Alfred-Nobel-Straße 50
40789 Monheim am Rhein (DE)**

(56) References cited:
**WO-A1-2017/096072     WO-A2-2007/133942
US-A1- 2010 331 779**

**Description**

**BACKGROUND OF THE DISCLOSURE**

**Field of the Disclosure**

The present disclosure is directed to fluid delivery applications and, particularly, to a fluid injector system configured to remove gas from at least one fluid reservoir thereof. The present disclosure is further directed to a method of gas removal from at least one fluid reservoir and a computer program product for executing the method.

**Description of the Related Art**

In many medical diagnostic and therapeutic procedures, a medical practitioner, such as a physician or radiologist, injects a patient with a fluid. In recent years, a number of injector-actuated syringes and powered injectors for pressurized injection of fluids have been developed for use in procedures such as angiography, computed tomography (CT), and magnetic resonance imaging (MRI). In these procedures, a fluid, such as a contrast agent, may be used to highlight certain internal organs or portions of the body during an imaging process. Meanwhile, saline, or a similar flushing agent, may be used to ensure complete injection of the bolus of the contrast agent.

When drawing a fluid, such as those mentioned above, into a fluid injector system, air or gas bubbles may adhere to the inner surfaces of the system. Under conventional conditions, it can be difficult to remove these air bubbles prior to injection. Typically, the quantities of air are sufficiently small and do not present a concern if injected into the vasculature of the patient. However, there are instances where injection of air, even in low volumes, may be harmful. For example, the injection of air into a vein or artery, for example during an angiography procedure, may cause an air embolism. Even small quantities of air, which may not present the concern of an air embolism, may result in imaging artifacts which may degrade the diagnostic efficacy of an imagining procedure. Further, the presence of air within a fluid injector system may result in transient fluid dynamics, such as a change in flow rate or pressure within the system. These changes within the fluid injector system may lead to further complications, such as inaccurate amounts of fluid being delivered to the patient. The presence of air bubbles within the fluid injector system may also lead the patient or technician to have a negative perception of the injection. Therefore, removing air from a fluid injector system may be advantageous not only to the injection procedure itself, but also to a patient's perception of the procedure.

Various methods how to remove air from medical fluid injectors or medical fluid lines have are known:
US 2010/0331779 A1 discloses a method of removing air from a flow path of a medical fluid injection system. An exemplary method performed by the medical fluid injection device includes delivering a first amount of fluid to a fluid flow path, isolating fluid flow along the flow path, forming a vacuum condition upstream of the fluid isolation, re-establishing fluid communications along the flow path, and delivering a second amount of fluid to the flow path.

WO 2017/096072 A1 discloses an example of a method of enabling medical fluid transfer between a source container and a destination container. The method comprises the steps of providing a closed-system fluid transfer module. The module comprises a first closeable, resealable medical connector and a second closeable, resealable medical connector, a multidirectional fluid control valve with a driving interface configured to interface with an electro-mechanical driver of an electronic medical fluid transfer device, and an intermediate container or an intermediate pumping region. The user is instructed to couple the closed-system fluid transfer module to the electronic medical fluid transfer device.

WO 2007/133942 A2 discloses a method to purge air from a pressurizing unit that is used in a powered fluid injection system. The method includes moving a drive mechanism in a forward direction to an extended position within a pressurizing unit, retracting the drive mechanism in a rearward direction to partially fill the pressurizing unit with a first quantity of medical fluid, moving the drive mechanism in the forward direction to remove air from the pressurizing unit, and retracting the drive mechanism in the rearward direction to fill the pressurizing unit with a second quantity of fluid, the second quantity of fluid being greater than the first quantity of fluid.

However, the prior art does not teach an injector first determining the amount of actuator displacement necessary to create a vacuum sufficient to dislodge air bubbles prior to implementing a purge protocol.

**SUMMARY OF DISCLOSURE**

In view of the disadvantages of injecting air into a patient, there is a need in the art for improved methods of gas removal from fluids in fluid reservoirs of a fluid injector system. The present disclosure is generally directed to systems, methods, and computer program products for removing gas from at least one fluid reservoir of a fluid injector system.

According to various aspects of the present disclosure, a fluid injector system includes at least one actuator configured to change the internal volume of the at least one fluid reservoir, at least one fluid reservoir having at least one interior surface and defining an internal volume, and at least one processor. The at least one processor is programmed or configured to drive the actuator to at least partially fill the at least one fluid reservoir with a fluid from a fluid source, drive the actuator to generate an at least a partial vacuum within the internal volume to dislodge one or more gas bubbles adhered to the at least one interior surface and to cause the one or more gas bubbles to coalesce into a coalesced bubble, and drive the actuator to expel the coalesced bubble from an outlet of the at least one fluid reservoir. According to some aspects of the present disclosure, the at least one processor is further programmed or configured to, prior to driving the actuator to generate at least the partial vacuum within the internal volume, close the outlet of the at least one fluid reservoir to fluidly isolate the internal volume. According to certain aspects, the at least one processor is further programmed or configured to, after closing the outlet of the at least one fluid reservoir, drive the actuator to pressurize the coalesced bubble. According to some aspects, the at least one processor is further programmed or configured to, prior to driving the actuator to expel the coalesced bubble from an outlet of the at least one fluid reservoir, open the outlet of the at least one fluid reservoir to provide fluid communication between the internal volume and one of the fluid source and a fluid outlet pathway. According to some aspects of the present disclosure, the at least one processor is further programmed or configured to vibrate, oscillate, or provide an impact force on at least one of the at least one interior surfaces to dislodge the one or more gas bubbles.

According to some aspects of the present disclosure, the at least one fluid reservoir includes a syringe. The actuator may include a piston configured to move reciprocally to change the internal volume of the syringe. The at least one interior surface may further include a surface of the plunger. According to some aspects, the syringe includes a rolling diaphragm syringe. The at least one interior surface includes an inner surface of the rolling diaphragm syringe. The piston is releasably connected to a proximal end wall of the syringe and is configured to reciprocally move the proximal end wall of the rolling diaphragm syringe.

According to some aspects of the present disclosure, driving the actuator to at least partially fill the at least one fluid reservoir includes moving a piston of the fluid injector system operatively connected to the at least one fluid reservoir in a first direction. Driving the actuator to generate the at least partial vacuum within the internal volume includes moving the piston in the first direction. Driving the actuator to expel the coalesced bubble includes moving the piston in a second direction opposite the first direction.

According to some aspects of the present disclosure, the at least one fluid reservoir may further include a valve in fluid communication with the outlet of the at least one fluid reservoir. The valve has at least a first open position and a second closed position. Closing the outlet of the at least one fluid reservoir includes moving the valve to the second closed position. According to some aspects, there is fluid communication between the internal volume of the at least one fluid reservoir and the fluid source when the valve is in the first open position. The valve may further include a third open position allowing fluid communication between the internal volume of the at least one fluid reservoir and a fluid outlet pathway.

According to some aspects of the disclosure, the at least partial vacuum generated within the internal volume is sufficient to extract at least a portion of a dissolved gas from the fluid. The dissolved gas that is extracted coalesces into the coalesced bubble.

According to some aspects of the present disclosure, the at least one processor is further programmed or configured to drive the actuator to prime a fluid path set in fluid communication with a fluid outlet pathway after the coalesced bubble is expelled from the at least one fluid reservoir. According to some aspects, the at least one processor is further programmed or configured to determine the amount of vacuum necessary to dislodge the one or more gas bubbles from the at least one interior surface based on at least one of a surface tension of the fluid, a surface tension of the gas, a surface texture of the at least one interior surface, interfacial surface energy between the at least one interior surface and the gas, a weight of the fluid above the one or more gas bubbles in the fluid reservoir, and a buoyancy of the one or more gas bubble in the fluid. According to some aspects, the at least one processor is further programmed or configured to measure the pressure within the internal volume of the fluid reservoir and adjust the at least partial vacuum within the internal volume based on the measured pressure. According to some aspects, adjusting the at least partial vacuum includes at least one of increasing or decreasing a speed of retraction of the actuator and increasing or reducing a diameter of at least a portion of a fluid path set in fluid communication with the internal volume of the fluid reservoir.

According to some aspects of the present disclosure, a method for removing gas bubbles from at least one fluid reservoir of a fluid injector system includes driving an actuator to at least partially fill the at least one fluid reservoir with a fluid from a fluid source, driving the actuator to generate an at least partial vacuum within an internal volume of the at least one fluid reservoir to dislodge one or more gas bubbles adhered to least one interior surface of the at least one fluid reservoir and to cause the one or more gas bubbles to coalesce into a coalesced bubble, and driving the actuator to expel the coalesced bubble from an outlet of the at least one fluid reservoir.

According to some aspects, the method may further include, prior to driving the actuator to generate at least the partial vacuum within the internal volume, closing the outlet of the at least one fluid reservoir to fluidly isolate the internal volume. According to some aspects, the method may further include, after closing the outlet of the at least one fluid reservoir, driving the actuator to pressurize the coalesced bubble.

According to some aspects, the method may further include, prior to driving the actuator to expel the coalesced bubble from an outlet of the at least one fluid reservoir, opening the outlet of the at least one fluid reservoir to provide fluid communication between the internal volume and one of the fluid source and a fluid outlet pathway.

According to some aspects of the present disclosure, driving the actuator to at least partially fill the at least one fluid reservoir includes moving a piston of the fluid injector system operatively connected to the at least one fluid reservoir in a first direction. Driving the actuator to generate the at least partial vacuum within the internal volume includes moving the piston in the first direction. Driving the actuator to expel the coalesced bubble includes moving the piston in a second direction opposite the first direction.

According to some aspects, the method may further include vibrating, oscillating, or providing an impact force on at least one of the at least one interior surfaces to dislodge the one or more gas bubbles.

According to some aspects of the present disclosure, closing the outlet of the at least one fluid reservoir includes moving a valve at the outlet from a first open position where the internal volume is in fluid communication with the fluid source to a second closed position where the internal volume is fluidly isolated from the fluid source. According to some aspects, there is fluid communication between the internal volume of the at least one fluid reservoir and the fluid source when the valve is in the first open position. The valve may further include a third open position allowing fluid communication between the internal volume of the at least one fluid reservoir and a fluid outlet pathway. According to some aspects, the method may further include driving the actuator to prime a fluid path set in fluid communication with reservoir fluid outlet pathway after the coalesced bubble is expelled from the at least one fluid reservoir.

According to some aspects of the present disclosure, the method may further include determining the amount of vacuum necessary to dislodge the one or more gas bubbles from the at least one interior surface based one at least one of a surface tension of the fluid, a surface tension of the gas, a surface texture of the at least one interior surface, and buoyancy of the one or more gas bubble in the fluid. According to some aspects, the method may further include measuring the pressure within the internal volume of the fluid reservoir and adjusting the at least partial vacuum within the internal volume based on the measured pressure. According to some aspects of the present disclosure, adjusting the at least partial vacuum includes at least one of: increasing or decreasing a speed of retraction of the actuator; and increasing or reducing a diameter of at least a portion of a fluid path set in fluid communication with the internal volume of the fluid reservoir.

According to some aspects of the present disclosure, a method for removing gas bubbles from at least one fluid filled fluid reservoir of a fluid injector system includes generating at least a partial vacuum within an internal volume of the at least one fluid reservoir dislodging one or more gas bubbles adhered to least one interior surface of the at least one fluid reservoir. The vacuum causes the one or more dislodged gas bubbles to enlarge and coalesce into a coalesced bubble. The method may further include expelling the coalesced bubble from an outlet of the at least one fluid reservoir. According to some aspects of the present disclosure, the method may further include closing the outlet of the at least one fluid reservoir prior to generating the at least partial vacuum within the internal volume.

According to some aspects, the method may further include pressurizing the coalesced bubble after closing the outlet of the at least one fluid reservoir. According to some aspects of the present disclosure, the method may further include opening the outlet of the at least one fluid reservoir prior to expelling the coalesced bubble from an outlet of the at least one fluid reservoir.

According to some aspects of the present disclosure, the at least one fluid reservoir may comprise a syringe. Generating the at least partial vacuum within an internal volume includes driving a piston of the fluid injection system in a first direction to increase the internal volume. Expelling the coalesced bubble including driving the piston of the fluid injection system in a second direction to decrease the internal volume.

According to some aspects of the present disclosure, the method may further include priming a fluid path set in fluid communication with the at least one fluid reservoir after the coalesced bubble is expelled from the at least one fluid reservoir.

According to some aspects of the present disclosure, generating a vacuum in the internal volume includes generating the at least partial vacuum sufficient to extract at least a portion of a dissolved gases from the fluid, and wherein the dissolved gas that is extracted coalesces into the coalesced bubble.

According to some aspects of the present disclosure, a computer program product includes at least one non-transitory computer-readable medium including program instructions for removing gas from at least one fluid reservoir of a fluid injector system. When executed by at least one processor, the program instructions cause the at least one processor to drive at least one actuator of the fluid injector system to at least partially fill the at least one fluid reservoir with a fluid from a fluid source, drive the at least one actuator to generate an at least partial vacuum within an internal volume of the at least one fluid reservoir to dislodge one or more gas bubbles adhered to an at least one interior surface of the at least one fluid reservoir and to cause the one or more gas bubbles to coalesce into a coalesced bubble, and drive the actuator to expel the coalesced bubble from an outlet of the at least one fluid reservoir. According to some aspects of the present disclosure, the instructions further cause the at least one processor to, prior to driving the actuator to generate the at least partial vacuum within the internal volume, close the outlet of the at least one fluid reservoir to fluidly isolate the internal volume. According to some aspects of the present disclosure, the instructions further cause the at least one processor to, after closing the outlet of the at least one fluid reservoir, drive the actuator to pressurize the coalesced bubble. According to some aspects of the present disclosure, the instructions further cause the at least one processor to vibrate, oscillate, or provide an impact force on at least one of the at least one interior surfaces to dislodge the one or more gas bubbles.

According to some aspects of the present disclosure, the instructions further cause the at least one processor to, prior to driving the actuator to expel the coalesced bubble from an outlet of the at least one fluid reservoir, open the outlet of the at least one fluid reservoir to provide fluid communication between the internal volume and one of the fluid source and a fluid outlet pathway.

According to some aspects of the present disclosure, the syringe includes a rolling diaphragm syringe. The at least one interior surface includes an inner surface of the rolling diaphragm syringe. The piston is releasably connected to a proximal end wall of the rolling diaphragm syringe and is configured to reciprocally move the proximal end wall of the rolling diaphragm syringe.

According to some aspects of the present disclosure, driving the actuator to at least partially fill the at least one fluid reservoir includes moving a piston of the fluid injector system operatively connected to the at least one fluid reservoir in a first direction. Driving the actuator to generate the at least partial vacuum within the internal volume includes moving the piston in the first direction. Driving the actuator to expel the coalesced bubble includes moving the piston in a second direction opposite the first direction.

According to some aspects of the present disclosure, the at least one fluid reservoir may further include a valve in fluid communication with the outlet of the at least one fluid reservoir. The valve has at least a first open position and a second closed position. Closing the outlet of the at least one fluid reservoir includes moving the valve to the second closed position. According to some aspects of the present disclosure, there is fluid communication between the internal volume of the at least one fluid reservoir and the fluid source when the valve is in the first open position. The valve may further include a third open position allowing fluid communication between the internal volume of the at least one fluid reservoir and a fluid outlet pathway.

According to some aspects of the present disclosure, the at least partial vacuum generated within the internal volume is sufficient to extract at least a portion of a dissolved gas from the fluid. The dissolved gas that is extracted coalesces into the coalesced bubble. According to some aspects of the present disclosure, the instructions further cause the at least one processor to drive the actuator to prime a fluid path set in fluid communication with a fluid outlet pathway after the coalesced bubble is expelled from the fluid reservoir.

According to some aspects of the present disclosure, the instructions further cause the at least one processor to determine the amount of vacuum necessary to dislodge the one or more gas bubbles from the at least one interior surface based on at least one of a surface tension of the fluid, a surface tension of the gas, a surface texture of the at least one interior surface, interfacial surface energy between the at least one interior surface and the gas, a weight of the fluid above the one or more gas bubbles in the fluid reservoir, and a buoyancy of the one or more gas bubbles in the fluid. According to some aspects of the present disclosure, the instructions further cause the at least one processor to measure the pressure within the internal volume of the fluid reservoir and adjust the at least partial vacuum within the internal volume based on the measured pressure.

According to some aspects of the present disclosure, adjusting the at least partial vacuum includes at least one of increasing or decreasing a speed of retraction of the actuator and increasing or reducing a diameter of at least a portion of a fluid path set in fluid communication with the internal volume of the fluid reservoir.

These and other features and characteristics of fluid injector systems, as well as computer program products and methods of operation and functions of the related elements of structures and the combination of parts and economies of manufacture, will become more apparent upon consideration of the following description and the appended claims with reference to the accompanying drawings, all of which form a part of this specification. It is to be expressly understood, however, that the drawings are for the purpose of illustration and description only. The invention is defined by the appended claims.

## BRIEF DESCRIPTION OF THE DRAWINGS

FIG. 1 is a perspective view of a multi-fluid delivery system, according to one example of the present disclosure;
FIG. 2 is a perspective view of the multi-patient disposable set (MUDS) within the multi-fluid delivery system of FIG. 1;
FIG. 3A is a perspective view of a connection interface prior to connecting a single-use disposable set (SUDS) connector with a multi-fluid delivery system;

**FIG. 3B** is a perspective view of the connection interface of **FIG. 3A** showing the SUDS connector connected with the multi-fluid delivery system;

**FIG. 4** is a schematic view of an electronic control system of a multi-fluid delivery system according to examples herein;

**FIG. 5** is a perspective view of a multi-fluid delivery system, according to examples of the present disclosure;

**FIG. 6A** is a side cross-sectional view of a syringe according to examples of the present disclosure with the syringe shown in an unrolled state;

**FIG. 6B** is a side cross-sectional view of the syringe of **FIG. 6A** with the syringe shown in a rolled state;

**FIG. 7** is a flowchart of a method for gas removal from a closed fluid reservoir according to examples of the present disclosure;

**FIG. 8A-8B** are schematic illustrations of an at least partial vacuum being placed on a closed fluid reservoir according to examples of the present disclosure;

**FIG. 8C** is a schematic illustration of the at least partial vacuum being placed on a closed fluid reservoir of **FIGS. 8A-8B,** with a coalesced gas bubble formed therein;

**FIG. 9** is a graphical representation of the pressure in a fluid reservoir versus time in an example of the present disclosure;

**FIGS. 10A-10B** are illustrations of open fluid reservoirs in examples of the present disclosure;

**FIG. 11** is a flowchart of a method for gas removal from an open fluid reservoir in an example of the present disclosure;

**FIGS. 12A-12B** are illustrations of an at least partial vacuum being placed on an open fluid reservoir by varying piston retraction speed, in an example of the present disclosure;

**FIGS. 13A-13B** are illustrations of an at least partial vacuum being placed on an open fluid reservoir by altering fluid path diameter, in an example of the present disclosure;

**FIGS. 14A-14B** are illustrations of fluid reservoirs having a gas collection chamber in examples of the present disclosure;

**FIG. 15** is an illustration of a fluid reservoir having a gas collection chamber in an example of the present disclosure;

**FIG. 16** is a graphical representation of bubble size versus vacuum pressure necessary to dislodge the bubbles in an example of the present disclosure; and

**FIG. 17** is a graphical representation of retraction of the piston versus vacuum pressure necessary to dislodge the bubbles in an example of the present disclosure.

## DETAILED DESCRIPTION

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures. When used in relation to a syringe of a multi-patient disposable set, the term "proximal" refers to a portion of a syringe nearest a piston for delivering fluid from a syringe. Spatial or directional terms, such as "left", "right", "inner", "outer", "above", "below", and the like, are not to be considered as limiting as the various features can assume various alternative orientations. All numbers used in the specification and claims are to be understood as being modified in all instances by the term "about". The term "about" means a range of plus or minus ten percent of the stated value.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

As used herein, the term "at least a partial vacuum" means a reduction of the pressure inside the fluid reservoir relative to the pressure outside the fluid reservoir. For example, if the outside of the fluid reservoir is at atmospheric pressure and the interior of the fluid reservoir is depressurized to at least a partial vacuum, the interior of the fluid reservoir may be at a reduced pressure of at least 0.1 atm less than the exterior of the fluid reservoir, in other embodiments at least 0.25 atm less than the exterior pressure of the fluid reservoir, and in other embodiments at a reduced pressure of at least 0.5 atm less than the exterior of the fluid reservoir. As used herein, specific values for pressure refer to gauge pressure unless otherwise noted. For example, a pressure of 0 atm or 0 psi corresponds to standard atmospheric pressure (i.e. 1 atm or 14.7 psi absolute pressure). As used herein, the term "air" is used synonymously with "gas" and can mean any gas bubble or any gas dissolved in fluid.

It is also to be understood that the specific devices and processes illustrated in the attached drawings, and described in the following specification, are simply exemplary examples of the disclosure. Hence, specific dimensions and other physical characteristics related to the examples disclosed herein are not to be considered as limiting.

When used in relation to a fluid reservoir, such as a syringe, a rolling diaphragm or single-use disposable set connector, the term "distal" refers to a portion of the fluid reservoir nearest to a patient. When used in relation to a fluid reservoir, such as a syringe, a rolling diaphragm or single-use disposable set connector, the term "proximal" refers to a portion of the fluid reservoir nearest to the injector system.

The term "open", when used to refer to a fluid delivery component, means that the system is in fluid connection with an outlet to atmospheric pressure, for example through a nozzle or the open end of a tubing component or catheter. In an open system, fluid flow may be constrained or restricted, for example by forcing a fluid through a small diameter fluid path where flow may be determined by physical parameters of the system and the fluid, such as tubing diameter, fluid path constrictions, applied pressure, viscosity, etc. The term "closed" or "closeable", when used to refer to a fluid delivery component, means that the system has at least one state in which the component is not in fluid connection with an outlet under atmospheric pressure or the fluid in the fluid reservoir is fluidly isolated, for example where fluid flow is stopped by a valve, such as a stopcock, high crack pressure valve, pinch valve, and the like, that closes a fluid pathway. As used herein, the phrase "at least partial vacuum" means a gauge pressure less than the current atmospheric pressure, for example from -14.7 psi to -0.1 psi.

Referring to the drawings in which like reference characters refer to like parts throughout the several views thereof, one embodiment of the present disclosure is generally directed to a multi-fluid medical injector/injector system **100** (hereinafter "fluid injector system **100**") having a multi-patient disposable set (MUDS) **130** configured for delivering fluid to a patient using a single-use disposable set (SUDS) **190** connector. The fluid injector system **100** includes multiple components as individually described herein. Generally, the fluid injector system **100** has a powered injector or other administration device and a fluid delivery set intended to be associated with the injector to deliver one or more fluids from one or more multi-dose containers under pressure into a patient, as described herein. The various devices, components, and features of the fluid injector system **100** and the fluid delivery set associated therewith are likewise described in detail herein. While the various embodiments of the methods and processor are shown with reference to an injector system having a MUDS and SUDS configuration, the disclosure is not limited to such an injector system and may be utilized in other syringe based injector systems, such as but not limited to those described in U.S. Patent Nos. 7,553,294, 7,563,249, 8,945,051, 9,173,995, 10,124,110; and U.S. Application Serial Nos. 15/305,285, 15/541,573, 15/568,505.

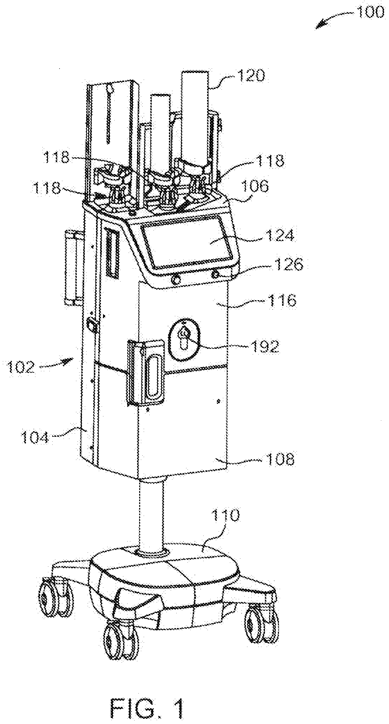

With reference to **FIG. 1,** a fluid injector system **100** according to one embodiment includes an injector housing **102** having opposed lateral sides **104,** a distal or upper end **106,** and a proximal or lower end **108.** The housing **102** encloses the various mechanical drive components, electrical and power components necessary to drive the mechanical drive components, and control components, such as electronic memory and electronic control devices (hereinafter electronic control device(s)), used to control operation of reciprocally movable pistons **103** associated with the fluid injector system **100** described herein. Such pistons **103** may be reciprocally operable via electro-mechanical drive components such as a ball screw shaft driven by a motor, a voice coil actuator, a rack-and-pinion gear drive, a linear motor, and the like. In some examples, at least some of the mechanical drive components, electrical and power components, and control components may be provided on the base **110.**

With continued reference to **FIG. 1,** the fluid injector system **100** may have at least one door **116** that encloses at least some of the MUDS, the mechanical drive components, electrical and power components, and control components.

The fluid injector system **100** may include at least one bulk fluid connector **118** for connection with at least one bulk fluid source **120.** In some examples, a plurality of bulk fluid connectors **118** may be provided. For example, as shown in the fluid injector embodiment illustrated in **FIG. 1,** three bulk fluid connectors **118** may be provided in a side-by-side or other arrangement. In some examples, the at least one bulk fluid connector **118** may be a spike configured for removably connecting to the at least one bulk fluid source **120,** such as a vial, a bottle, or a bag. The at least one bulk fluid connector **118** may be formed on the multi-patient disposable set, as described herein. The at least one bulk fluid source **120** may be configured for receiving a medical fluid, such as saline, contrast solution, or other medical fluid, for delivery to the fluid injector system **100.**

With continued reference to **FIG. 1,** the fluid injector system **100** includes one or more user interfaces **124,** such as a graphical user interface (GUI) display window. The user interface **124** may display information pertinent to a fluid injection procedure involving fluid injector system **100,** such as injection status or progress, current flow rate, fluid pressure, and volume remaining in the at least one bulk fluid source **120** connected to the fluid injector system **100** and may be a touch screen GUI that allows an operator to input commands and/or data for operation of fluid injector system **100.** While the user interface **124** is shown on the injector housing **102,** such user interface **124** may also be in the form of, or the fluid injector system **100** may additionally have, a remote display that is wired or wirelessly linked to the housing **102** and control and mechanical elements of fluid injector system **100,** for example in a remote room designed to shield the user from exposure to x-rays. In some examples, the user interface **124** may be a tablet computer that is detachably connected to the housing **102** and is in wired or wirelessly linked communication with the housing **102.** Additionally, the fluid injector system **100** and/or user interface **124** may include at least one control button **126** for tactile operation by an attendant operator of the fluid injector system **100.** In certain examples, the at least one control button **126** may be part of a keyboard for inputting commands and/or data by the operator. The at least one control button **126** may be hard-wired to the electronic control device(s) associated with the fluid injector system **100** to provide direct input to the electronic control device(s). The at least one control button **126** may also be a graphical part of the user interface **124,** such as a touch screen. In either arrangement, the at least one control button **126** desirably provides certain individual control features to the attendant operator of the fluid injector system **100,** such as, but not limited to: (1) acknowledging that a multi-patient disposable set has been loaded or unloaded; (2) selecting or programing an injection protocol; (3) filling/purging of the fluid injector system **100;** (4) inputting information and/or data related to the patient and/or injection procedure; (5) preloading the fluid injector system **100;** and (6) initiating/stopping an injection procedure. The user interface **124** and/or any electronic processing units associated with the fluid injector system **100** may be wired or wirelessly connected to an operation and/or data storage system such as a hospital network system.

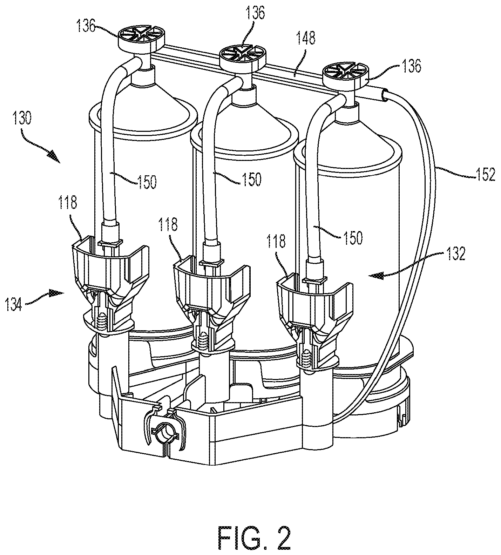

With reference to **FIG. 2,** the fluid injector system **100** includes a MUDS **130** that is removably connected to the fluid injector system **100** for delivering one or more fluids from the one or more bulk fluid sources **120** to the patient. Examples and features of embodiments of the MUDS are further described in PCT International Application No. PCT/US2016/012434.

The MUDS **130** may include one or more fluid reservoirs **132,** such as one or more syringes. As used herein, the term "fluid reservoir" means any container capable of taking in and delivering a fluid, for example during a fluid injection procedure including, for example a syringe, a rolling diaphragm, a pump, a compressible bag, and the like. Fluid reservoirs may include the interior volume of at least a portion of a fluid pathway, such as one or more tubing lengths, that are in fluid communication with the interior of the fluid reservoir, including fluid pathway portions that remain in fluid communication with the fluid reservoir after the system is closed or fluidly isolated from the remainder of the fluid pathway. In some examples, the number of fluid reservoirs **132** may correspond to the number of bulk fluid sources **120.** For example, with reference to **FIG. 2,** the MUDS **130** has three syringes **132** in a side-by-side arrangement such that each syringe **132** is fluidly connectable to one or more of the corresponding three bulk fluid sources **120.** In some examples, one or two bulk fluid sources **120** may be connected to one or more syringes **132** of MUDS **130.** Each syringe **132** may be fluidly connectable to one of the bulk fluid sources **120** by a corresponding bulk fluid connector **118** and an associated MUDS fluid path **134.** MUDS fluid path **134** may have a spike element that connects to bulk fluid connector **118.**

With reference to **FIG. 2,** the MUDS **130** is removably connectable to the housing **102** of the fluid injector system **100.** As will be appreciated by one having ordinary skill in the art, it may be desirable to construct at least a portion of the MUDS **130** from a clear medical grade plastic in order to facilitate visual verification that a fluid connection has been established with the fluid injector system **100** or that air has been removed from the fluid reservoir. Visual verification is also desirable for confirming that no air bubbles are present within various fluid connections, for example after performing an air removal protocol, such as described herein. Various optical sensors (not shown) may also be provided to detect air either in the fluid lines or the fluid reservoir during a priming operation. Additionally, various lighting elements (not shown), such as light emitting diodes (LEDs), may be provided to actuate one or more optical sensors and indicate that sufficient air has been removed from the fluid reservoir.

With reference to **FIG. 2,** the MUDS **130** may include one or more valves **136,** such as stopcock valves, for controlling which medical fluid or combinations of medical fluids are withdrawn from the multi-dose bulk fluid source **120** (see **FIG. 1**) into the fluid reservoirs **132** and/or are delivered to a patient from each fluid reservoir **132.** In some examples, the one or more valves **136** may be provided on a distal end of the plurality of syringes **132** or on a manifold **148.** The manifold **148** may be in fluid communication via valves **136** and/or syringes **132** with a first end of the MUDS fluid path **134** that connects each syringe **132** to the corresponding bulk fluid source **120.** The opposing second end of the MUDS fluid path **134** may be connected to the respective bulk fluid connector **118** that is configured for fluidly connecting with the bulk fluid source **120.** Depending on the position of the one or more valves **136,** fluid may be drawn into the one or more syringes **132** or it may be delivered from the one or more syringes **132.** In a first position, such as during the filling of the syringes **132,** the one or more valves **136** are oriented such that fluid flows from the bulk fluid source **120** into the desired syringe **132** through a fluid inlet line **150,** such as a MUDS fluid path. During the filling procedure, the one or more valves **136** are positioned such that fluid flow through one or more fluid outlet lines **152** or manifold **148** is blocked. In a second position, such as during a fluid delivery procedure, fluid from one or more syringes **132** is delivered to the manifold **148** through the one or more fluid outlet lines **152** or syringe valve outlet ports. During the delivery procedure, the one or more valves **136** are positioned such that fluid flow through one or more fluid inlet lines **150** is blocked. In a third position, the one or more valves **136** are oriented such that fluid flow through the one or more fluid inlet lines **150** and the one or more fluid outlet lines **152** is blocked. Thus, in the third position, each of the one or more valves **136** isolates the corresponding syringe **132** and prevents fluid flow into and out of the corresponding syringe **132.** As such, each of the one or more syringes **132** and any portion of the manifold **148** between that syringe **132** and corresponding valve **136** defines a closed system. According to various aspects, the methods described herein may be used when fluid reservoir **132** is in the third, closed position

The one or more valves **136,** fluid inlet lines **150,** and/or fluid outlet lines **152** may be integrated into the manifold **148.** The one or more valves **136** may be selectively positioned to the first or second position by manual or automatic handling. For example, the operator may position the one or more valves **136** into the desired position for filling, fluid delivery, or the closed position. In other examples, at least a portion of the fluid injector system **100** is operable for automatically positioning the one or more valves **136** into a desired position for filling, fluid delivery, or the closed position based on input by the operator or by a protocol in the system controller, as described herein.

In some examples, the fluid outlet line **152** may also be connected to a waste reservoir on the fluid injector system **100**. In some examples, the waste reservoir is configured to receive waste fluid and air containing fluid expelled from the syringes **132** during, for example, a flushing, priming, air removal, or preloading operation.

Having generally described the components of the fluid injector system **100** and the MUDS **130,** the structure and method of use of a single-use disposable set **190** (SUDS) and its interaction with MUDS **130** will now be described.

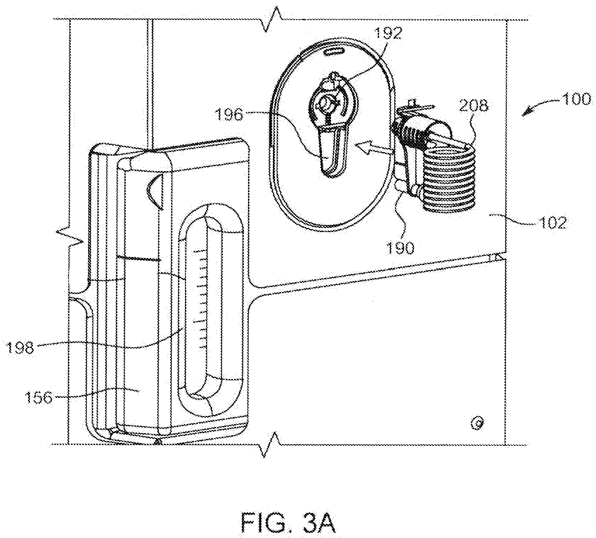

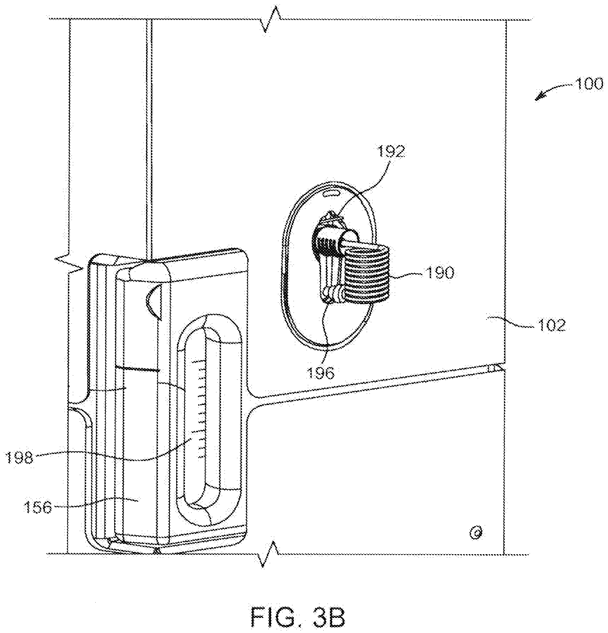

With reference to **FIGS. 3A and 3B,** the fluid injector system **100** has a connection port **192** that is configured to form a releasable fluid connection with at least a portion of the SUDS **190**. In some examples, the connection port **192** may be formed on the MUDS **130**. As described herein, the SUDS **190** may be connected to the connection port **192,** formed on at least a portion of the MUDS **130** and/or the housing **102**. Desirably, the connection between the SUDS **190** and the connection port **192** is a releasable connection to allow the SUDS **190** to be selectively disconnected from the connection port **192 (FIG. 3A)** and connected to the connection port **192 (FIG. 3B)**. In some examples, the SUDS **190** may be disconnected from the connection port **192** and disposed after each fluid delivery procedure, and a new SUDS **190** may be connected to the connection port **192** for a subsequent fluid delivery procedure.

Other examples and features of the SUDS **190** are described in U.S. Patent Publication No. 2016/0331951, filed July 7, 2016 and entitled "Single-Use Disposable Set Connector".

Having generally described the components of the fluid injector system **100,** the MUDS **130,** and the SUDS **190,** a method of operation of using the SUDS **190** will now be described in detail. In use, a medical technician or user removes the disposable SUDS **190** from its packaging (not shown) and inserts the fluid inlet port **202** into the connection port **192** on the MUDS **130**. The SUDS **190** may be secured to the MUDS **130** by inserting the locking tab **216** into the receiving slot **217** on the MUDS **130** and the controller determines that the SUDS **190** is securely connected to the MUDS **130,** for example as sensed by the sensor **242**. The fluid injector system **100** may perform an automatic priming or flushing operation for removing air from the MUDS **130** and the SUDS **190**. Prior to or as part of the automatic priming and/or flushing operation, removal of additional air or gas bubbled adhered to an interior surface of the fluid reservoir **132** that are not may be removed during a bulk air priming or flushing operation may be removed according to various embodiments described herein. During such priming or flushing operations, fluid from the MUDS **130** including any air that was entrapped or present in the fluid reservoir **132** is injected through the connection port **192** and into the tubing **208** of the SUDS **190**. The fluid flows through the tubing **208,** the connector **214** and through the waste outlet port **204** and into the waste reservoir **156**. Once the automatic priming or flushing operation is completed, the tubing **208** may optionally be preloaded by injecting fluid from the MUDS **130** through the connection port **192.** After the automatic priming or flushing operation, including the air or gas removal processes described herein, and, optionally, the preloading operation are completed, the medical technician disconnects the connector **214** from the waste outlet port **204.** The connector **214** may then be connected to the patient via a catheter, vascular access device, needle, or additional fluid path set to facilitate fluid delivery to the patient. Once the fluid delivery is completed, the SUDS **190** is disconnected from the patient and the MUDS **130** by disengaging the locking tab **216** of the SUDS **190** from the receiving slot **217** on the MUDS **130.**

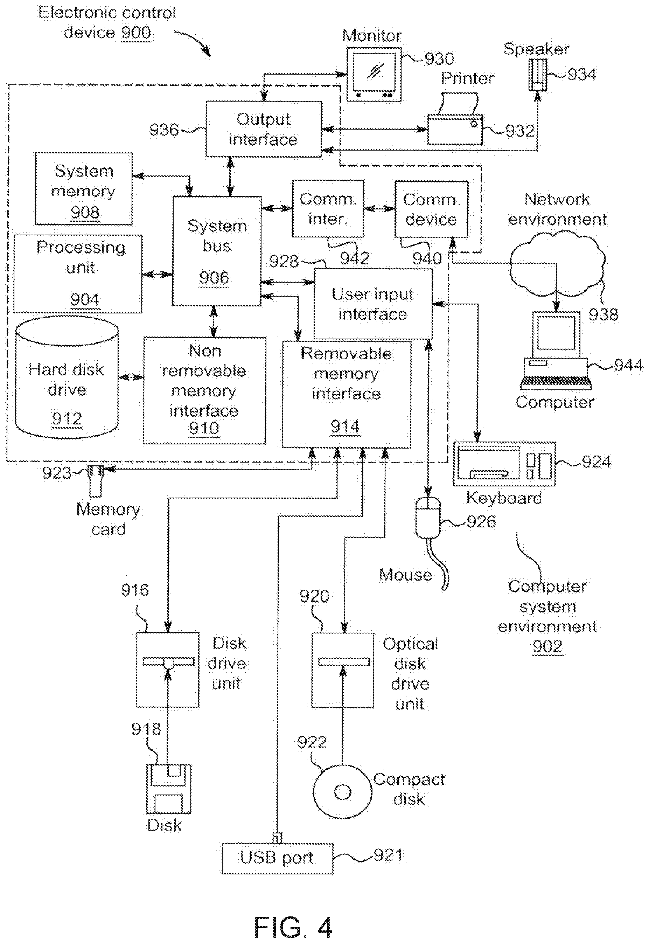

With reference to **FIG. 4,** an electronic control device **900** may be associated with fluid injector system **100** to control the filling and delivery operations. In some examples, the electronic control device **900** may control the operation of various valves, stopcocks, piston members, and other elements to affect a desired gas/air removal, filling, and/or delivery procedure. For example, the electronic control device **900** may include a variety of discrete computer-readable media components. For example, this computer-readable media may include any media that can be accessed by the electronic control device **900,** such as volatile media, non-volatile media, removable media, non-removable media, transitory media, non-transitory media, etc. As a further example, this computer-readable media may include computer storage media, such as media implemented in any method or technology for storage of information, such as computer-readable instructions, data structures, program modules, or other data; random access memory (RAM), read-only memory (ROM), electrically erasable programmable read-only memory (EEPROM), flash memory, cloud storage media, or other memory technology; solid state memory, cloud memory, CD-ROM, digital versatile disks (DVDs), or other optical disk storage; magnetic cassettes, magnetic tape, magnetic disk storage, or other magnetic storage devices; or any other medium which can be used to store the desired information and which can be accessed by the electronic control device **900.** Further, this computer-readable media may include communications media, such as computer-readable instructions, data structures, program modules, or other data in a modulated data signal, such as a carrier wave or other transport mechanism and include any information delivery media, wired media (such as a wired network and a direct-wired connection), and wireless media (such as acoustic signals, radio frequency signals, optical signals, infrared signals, biometric signals, bar code signals, etc.). Of course, combinations of any of the above should also be included within the scope of computer-readable media.

Electronic control device **900** may further include a system memory **908** with computer storage media in the form of volatile and non-volatile memory, such as ROM and RAM. A basic input/output system (BIOS) with appropriate computer-based routines assists in transferring information between components within electronic control device **900** and is normally stored in ROM. The RAM portion of system memory **908** may contain data and program modules that are immediately accessible to or presently being operated by a processor **904,** e.g., an operating system, application programming interfaces, application programs, program modules, program data, or other instruction-based device-readable codes.

With continued reference to **FIG. 4,** the electronic control device **900** may also include other removable or non-removable, volatile or non-volatile, transitory or non-transitory computer storage media products. For example, the electronic control device **900** may include a non-removable memory interface **910** that communicates with and controls a hard disk drive **912,** e.g., a non-removable, non-volatile magnetic medium; and a removable, non-volatile memory interface **914** that communicates with and controls a magnetic disk drive unit **916** (which reads from and writes to a removable, non-volatile magnetic disk **918),** an optical disk drive unit **920** (which reads from and writes to a removable, non-volatile optical disk **922,** such as a CD ROM), a Universal Serial Bus (USB) port **921** for use in connection with a removable memory card, etc. However, it is envisioned that other removable or non-removable, volatile or non-volatile computer storage media can be used in an exemplary computing system environment **902,** including, but not limited to, magnetic tape cassettes, DVDs, digital video tape, solid state RAM, solid state ROM, etc. These various removable or non-removable, volatile or non-volatile magnetic media are in communication with the processor **904** and other components of the electronic control device **900** via a system bus **906.** The drives and their associated computer storage media, discussed herein and illustrated in **FIG. 4,** provide storage of operating systems, computer-readable instructions, application programs, data structures, program modules, program data, and other instruction-based, computer-readable code for the electronic control device **900** (whether duplicative or not of this information and data in the system memory **908).**

A user may enter commands, information, and data into the electronic control device **900** through certain attachable or operable input devices, such as the user interface **124** shown in **FIG. 1,** via a user input interface **928.** A variety of such input devices may be utilized, e.g., a microphone, a trackball, a joystick, a touchpad, a touch-screen, a scanner, etc., including any arrangement that facilitates the input of data and information to the electronic control device **900** from an outside source. As discussed, these and other input devices are often connected to the processor **904** through the user input interface **928** coupled to the system bus **906,** but may be connected by other interface and bus structures, such as a parallel port, game port, or a USB. Still further, data and information can be presented or provided to a user in an intelligible form or format through certain output devices, such as a monitor **930** (to visually display this information and data in electronic form), a printer **932** (to physically display this information and data in print form), a speaker **934** (to audibly present this information and data in audible form), etc. All of these devices are in communication with electronic control device **900** through an output interface **936** coupled to system bus **906.** Any such peripheral output devices may be used to provide information and data to the user.

Electronic control device **900** may operate in a network environment **938** through the use of a communications device **940,** which is integral to electronic control device **900** or remote therefrom. Communications device **940** is operable by and in communication with the other components of the electronic control device **900** through a communications interface **942.** Using such an arrangement, the electronic control device **900** may connect with or otherwise communicate with one or more remote computers, such as a remote computer **944,** which may be a personal computer, a server, a router, a network personal computer, a peer device, or other common network nodes, and typically includes at least some of the components described in connection with the electronic control device **900.** Using appropriate communication devices **940,** e.g., a modem, a network interface or adapter, etc., computer **944** may operate within and communicate through a local area network (LAN) and a wide area network (WAN), but may also include other networks such as a virtual private network (VPN), an office network, an enterprise network, an intranet, the Internet, etc.

As used herein, the electronic control device **900** includes or is operable to execute appropriate custom-designed or conventional software to perform and implement the processing steps of the method and system of the present disclosure, thereby forming a specialized and particular computing system. Accordingly, the method and system may include one or more electronic control devices **900** or similar computing devices having a computer-readable storage medium capable of storing computer-readable program code or instructions that cause the processor **904** to execute, configure, or otherwise implement the methods, processes, and transformational data manipulations discussed hereinafter in connection with the present disclosure. Still further, the electronic control device **900** may be in the form of a personal computer, a personal digital assistant, a portable computer, a laptop, tablet, a palmtop, a mobile device, a mobile telephone, a server, or any other type of computing device having the necessary processing hardware to appropriately process data to effectively implement the computer-implemented method and system.

It will be apparent to one skilled in the relevant arts that the system may utilize databases physically located on one or more computers which may or may not be the same as their respective servers. For example, programming software on electronic control device **900** can control a database physically stored on a separate processor of the network or otherwise.

In some examples, the electronic control device **900** may be programmed so that automatic refill occurs based upon a preprogrammed trigger minimum volume in the respective syringes **132.** For example, when the volume of fluid remaining in at least one of the syringes **132** is less than a programmed volume, a syringe refill procedure is automatically initiated by the electronic control device **900.** The electronic control device **900** associated with the fluid injector system **100** may determine that the preprogrammed trigger minimum volume has been reached by tracking the fluid volume dispensed from the respective syringes **132** during operation of the fluid injector system **100.** Alternatively, fluid level sensors may be incorporated into the fluid injector system **100** and inputs from these fluid level sensors may be provided to the electronic control device **900** so that the electronic control device **900** may determine when the preprogrammed trigger minimum volume has been reached in at least one of the syringes **132.** The fill volume and rate of refill can be preprogrammed in the electronic control device **900.** The automatic refill procedure can be stopped either automatically by the electronic control device **900** or may be manually interrupted. In addition, an automatic refill procedure may be initiated when, at the completion of a fluid injection procedure, there is not enough fluid in at least one of the syringes **132** to perform the next programmed fluid injection procedure. Once the automatic refill has been triggered, the fluid injector may run the air removal protocol according to embodiments described herein.

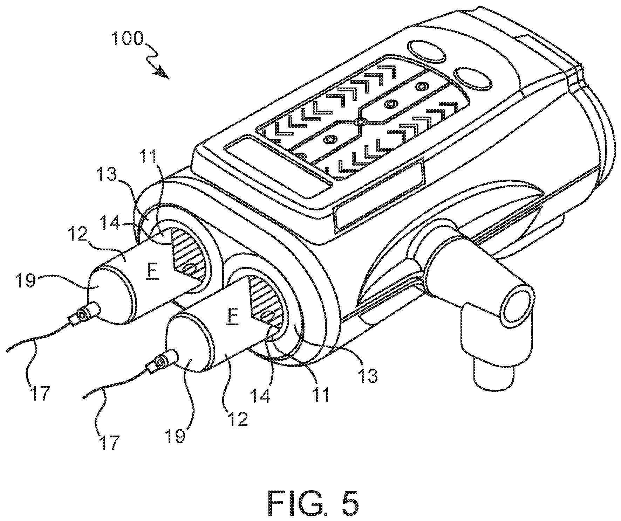

While **FIGS. 1-3B** illustrate one example of a fluid injector system **100** and associated components and structure, it is to be understood that the present disclosure is not limited to any particular type or variety of the fluid injector system **100.** Referring now to **FIG. 5,** another example of a fluid injector system **100** According to the present disclosure includes at least one fluid reservoir, such as syringe **12,** at least one piston **103** (not shown) connectable to at least one plunger **14,** and a fluid control module (not pictured). The at least one syringe **12** is generally adapted to interface with at least one component of the system, such as a syringe port **13.** The fluid injector system **100** is generally configured to deliver at least one fluid **F** to a patient during an injection procedure. The fluid injector system **100** is configured to releasably receive the at least one syringe **12,** which is to be filled with at least one fluid **F,** such as contrast media, saline solution, or any desired medical fluid. The system may be a multi-syringe injector, wherein several syringes may be oriented side-by-side or in another spatial relationship and are separately actuated by respective pistons associated with the injector. The at least one syringe **12** may be oriented in any manner such as upright, downright, or positioned at any degree angle.

With continued reference to **FIG. 5,** the injector system **100** may be used during a medical procedure to inject the medical fluid **F** into the body of a patient by driving a plunger **14** of at least one syringe **12** with a drive member, such as the at least one piston **103** (not shown). The at least one piston may be reciprocally operable upon at least a portion of the at least one syringe, such as the plunger **14.** Upon engagement, the at least one piston may move the plunger **14** toward the distal end **19** of the at least one syringe, as well as toward the proximal end **11** of the at least one syringe **12.**

A tubing set **17** may be in fluid communication with each syringe **12** to place each syringe in fluid communication with a catheter for delivering the fluid **F** from each syringes **12** to the catheter (not shown) inserted into a patient at a vascular access site. In certain embodiments, fluid flow from the one or more syringes **12** may be regulated by a fluid control module, which may be the same or similar to the electronic control device **900,** that operates various valves, stopcocks, and flow regulating structures to regulate the delivery of the at least one fluid to the patient based on user selected injection parameters, such as injection flow rate, duration, and total injection volume. The fluid control module is generally configured to perform various functions, those of which have the ability to aid in the removal of gas from the system, as will be described herein, along with other various embodiments.

In some examples, the fluid control module may instruct the fluid injector system 100 to fill the at least one syringe **12** with the at least one fluid **F.** The fluid injector system 100 may further include at least one bulk fluid source (not shown) for filling the at least one syringe **12** with fluid and in certain examples, the fluid injector system **100** may have a plurality of bulk fluid sources, one for each of the syringes **12,** for filling each of the syringes with the desired fluid. Filling the at least one syringe **12** with the at least one fluid **F** may be done by placing the at least one syringe **12** in fluid communication with at least one bulk fluid source and instructing the fluid injector system **100** to withdraw the piston, being removably engaged with the plunger **14** of the at least one syringe **12,** from the distal end **19** of the at least one syringe toward the proximal end **11** of the at least one syringe. Filling in such a manner provides that the at least one syringe may be oriented in any manner during the filling procedure, such as upwards, downwards, or at any degree angle. In certain embodiments, the fluid injector system **100** and fluid control module may be programmed to perform an air removal protocol, as described herein. In certain embodiments, the air removal protocol may be performed on an open system, i.e., the at least one syringe **12** may be in fluid connection with the tubing set **17** and the protocol may utilize one or more of the viscosity of the fluid, the flow rate, the difference between the diameter of the syringe and the flow path tubing to generate at least a partial vacuum, for example by quickly retracting the piston and plunger at a designated rate, to affect the air removal protocol as described herein. According to other embodiments, the distal end **19** of the syringe **12** and/or the tubing set **17** may include one or more valves or stop cocks to fluidly isolate the interior of the syringe from the outside to allow the injector system **100** to generate the at least partial vacuum sufficient to affect the air removal protocol, as described herein.

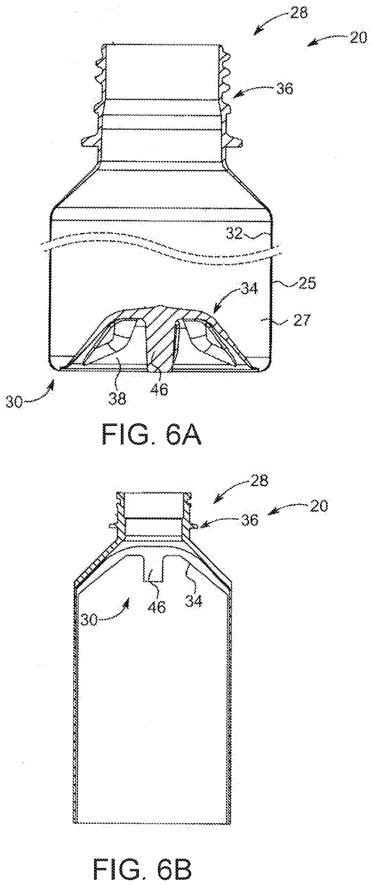

The examples of fluid injector systems **100** described in connection with **FIGS. 1-5** are generally illustrated as having rigid-bodied syringes **132, 12.** However, it is to be understood that the present disclosure contemplates that other fluid reservoirs and varieties of syringes may be utilized in the fluid injector systems **100** described herein. Referring now **FIG. 6A** and **6B,** in some examples a rolling diaphragm the syringe **20** generally includes a hollow body **25** defining an interior volume **27.** The body **25** has a forward or distal end **28,** a rearward or proximal end **30,** and a flexible sidewall **32** extending therebetween. The sidewall **32** of the syringe **20** defines a soft, pliable or flexible, yet self-supporting body that is configured to roll upon itself, as a rolling diaphragm, under the action of the piston **19.** In particular, the sidewall **32** is configured to roll such that its outer surface is folded and inverted in a radially inward direction as the piston **103** is moved in a distal direction **(FIG. 6B)** and unrolled and unfolded in the opposite manner in a radially outward direction as the piston **103** is retracted in a proximal direction **(FIG. 6A).**

The sidewall **32** may have a smooth, substantially uniform structure, or it may have one or more ribs provided thereon to facilitate the rollover during an injection procedure. According to these embodiments, the end wall **34** may have a piston engagement portion **46** located on a proximal end of the plunger to interact with the plurality of engagement elements of the various embodiments of the engagement mechanisms on a piston of the fluid injector. Examples of rolling diaphragm syringes and injector piston configurations suitable for use in the air removal protocols of the present disclosure are described in U.S. Application Publication Nos. 2017/0035974; and 2018/0261496; and in PCT International Application Publication Nos. WO 2018/075379; and WO 2018/0753 86.

With continued reference to **FIG. 6A-6B,** the rearward or proximal portion **30** of the sidewall **32** connects to a closed end wall **34,** and a forward or distal portion **28** of the sidewall **32** defines a discharge neck **36** opposite the closed end wall **34.** The closed end wall **34** may have a concave shape to facilitate the initiation of the inversion or rolling of the sidewall **32** and/or to provide a receiving pocket to receive a distal end of the piston **19.** For example, the closed end wall **34** may define a receiving end pocket **38** for interfacing directly with a similarly-shaped piston **19.**

Having described various aspects of the fluid injector system **100,** embodiments for processor programing and methods of gas removal from at least one fluid reservoir of the fluid injector system **100** will now be described. The at least one fluid reservoir may be as described herein and may, for example, include at least one of the syringes **132, 12** and/or the rolling diaphragm syringe **20.** Alternatively or in additionally, the at least one reservoir may include at least one bottle, or at least one collapsible bag. One of ordinary skill in the art would appreciate that this list is merely exemplary and the method of gas removal may extend to other reservoirs. Further, it should be appreciated that the method of gas removal can be performed on a fluid injector system of any fluid volume, such as for example on a fluid reservoir of 0.1 milliliters (mL) through 1000 mL and in other embodiments from 10 mL to 300 mL. As will be described in greater detail herein, the methods of gas removal According to the present disclosure relate to both closed and open fluid injector systems **100,** i.e., a fluid injector system where the one or more fluid reservoirs (optionally including at least a portion of the tubing set) may be in fluid communication with or fluidly isolated from the remainder of the fluid flow path of the system, including embodiments where at least one of the fluid reservoirs is in fluid isolation and at least one of the other reservoirs may be in fluid communication with the system.

In one example of the present disclosure, a method of gas removal may be utilized in a closed or closeable fluid injector system **100,** meaning that the fluid injector system **100** has at least one state in which the at least one fluid reservoir (optionally including at least a portion of the tubing set) is not exposed to atmospheric pressure and/or is fluidly isolated from the remainder of the fluid injection flow path. For example, with reference to the fluid injector system **100** of **FIGS. 1-3B,** each of the syringes **132** may be isolated from atmospheric pressure by rotating the associated valve **136** to a closed position to prevent fluid flow into and from the syringe **132.** The fluid injector system **100** of **FIG. 5** may also be converted into a closed or closeable system by placement of an isolation valve, such as a pinch valve, a shuttle valve, a check valve, high pressure crack valve, or a stopcock, in any of the fluid path sets **17** or between any of the fluid path sets **17** and the associated syringe **12.** One of ordinary skill in the art would appreciate that this list is merely exemplary and other types of isolation valves may be used.

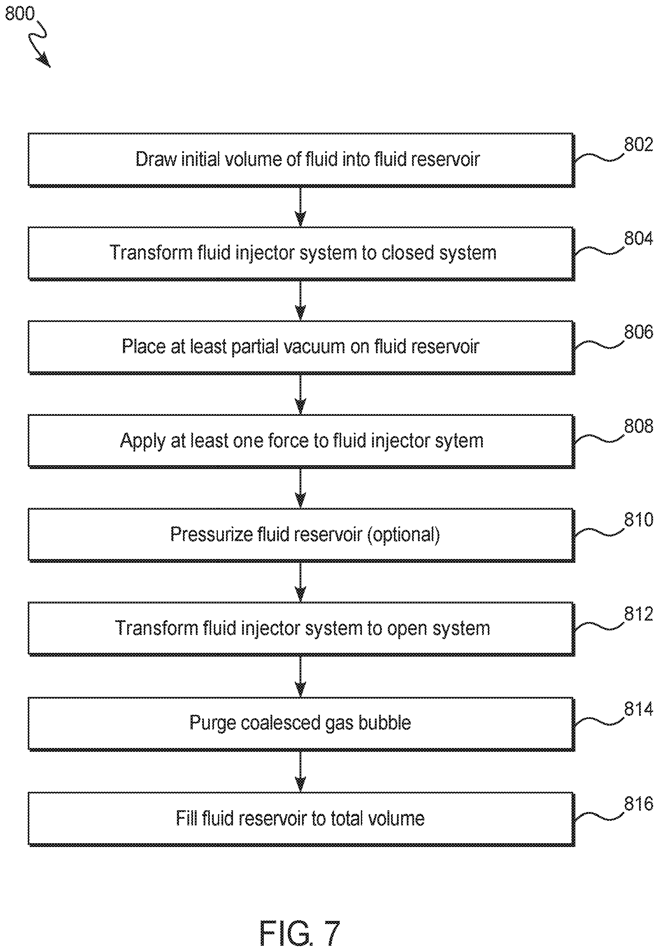

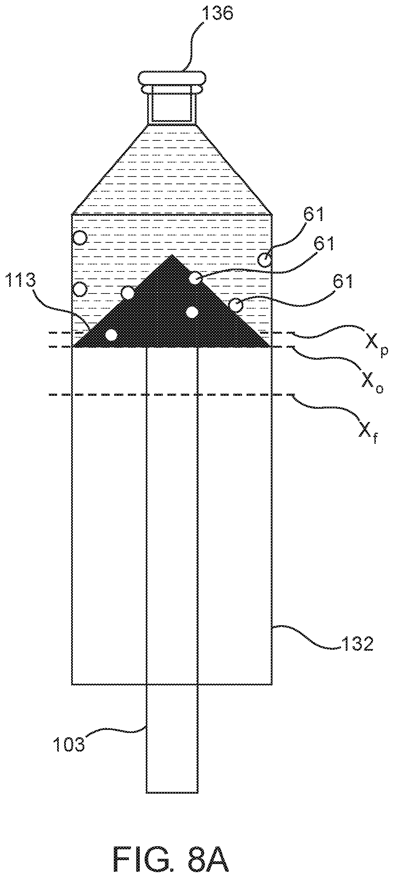

Referring now to **FIGS. 7** and **8A-8C,** a method **800** of gas removal from a closed or closeable system will be described. For simplicity, the method **800** will be described primarily with respect to the example of the fluid injector system **100** described in connection with **FIGS. 1-3B.** However, the method **800** may also be used with other fluid reservoir based fluid injector systems having a closable configuration that fluidly isolates the reservoir from the atmosphere, for example the fluid injector system **100** and associated syringes **12** illustrated in **FIG. 5,** having first converted the fluid injector system **100** of **FIG. 5** to a closeable system, or with a rolling diaphragm-based fluid injection system, as described herein. At step **802,** an initial volume of fluid is drawn into at least one fluid reservoir, e.g. at least one syringe **132,** of the fluid injector system **100.** The valve **136** associated with the at least one syringe **132** is placed in a position allowing fluid flow into the syringe **132** from a fluid source in fluid communication with the syringe **132,** such as one or more of the bulk fluid sources **120.** A plunger **113** positioned at a distal or near distal location with the syringe **132** may be moved proximally within the syringe **132** to draw an initial volume of fluid into the syringe **132.** In some examples, the plunger **113** may be removably connected to a distal end of the piston **103,** and the piston **103** may be actuated by the electronic control module **900** to move the plunger **113** within the syringe **132.** The initial volume of fluid drawn into the syringe **132** may be, for example, from 10 mL to 300 mL. The initial volume of fluid drawn into the syringe **132** may include air or other gas in a significant volume, the majority of which may be removed by a purge or prime sequence, and/or may have a clinically insignificant amount of air or gas adhered to one or more interior surfaces of the fluid reservoir, for example as small but visible bubbles. According to various embodiments, the small but visible bubbles may remain adhered to the one or more interior surfaces after a prime/purge sequence due to their size and/or buoyancy not being sufficient to overcome surface tension or adhesion between the bubble and the one or more interior surfaces. While potentially clinically insignificant, the gas may nevertheless be manifested as bubbles **61,** which may be visible to the patient and the technician through the transparent sidewall of the syringe **132.** In other procedures such as an angiography procedure, even small amounts of air or gas may be clinically significant and must be removed from the fluid reservoir prior to initiation of the injection procedure. The small bubbles **61** may particularly adhere to at least one interior surface of the syringe **132,** such as the sidewall of the syringe **132** and/or a surface of the plunger **113.** The bubbles **61** may manifest in a variety of sizes, with particularly large air bubbles **61** being up to 5 to 20 mL in volume or consisting of up to 10% of the volume of the syringe **132.** Smaller air bubbles **61** may be 0.001 to 5 mL in volume or consist of up to 2.5% of the volume of the syringe **132** may also be removed according to the present disclosure. Larger air bubbles may have sufficient buoyancy to overcome surface tension and adhesive forces with the interior surfaces and float to the distal end of the fluid reservoir to be removed during priming operations. However, as described herein, smaller air bubbles **61** may remain adhered to the interior surfaces during the priming/purging sequences, but may be removed utilizing the methods described herein.

With continued reference to **FIG. 7,** at step **804,** the fluid injector system **100** is transformed to a closed system, such that the at least one fluid reservoir is isolated from atmospheric pressure for example by closing an isolation valve. In some examples, the electronic control device **900** may be configured to transform the fluid injector system **100** from an open system to a closed system by rotating or otherwise adjusting the valve **136** to isolate the at least one fluid reservoir, e.g. at least one syringe **132,** from atmospheric pressure. In other examples, the valve **136** may be manually rotated to isolate the at least one syringe **132** from atmospheric pressure by a technician or a physician.

With continued reference to **FIGS. 7** and **8A-8B,** at step **806,** an at least partial vacuum may be placed on the at least one fluid reservoir, e.g. the at least one syringe **132.** The at least partial vacuum within the at least one fluid reservoir may be achieved by expanding the volume of the closed system, resulting in a pressure drop and thus creation of the at least partial vacuum. In some embodiments, the at least partial vacuum may be achieved by creating a displacement within the fluid injector system **100,** for example by reciprocally retracting the plunger **113** of the at least one syringe **132** by actuating the piston **103** via the electronic control module **900.** In some examples, the plunger **113** may be moved via the piston **103** at a constant speed, for example in a range of approximately 0 mL/s to 50 mL/s. In some examples, movement of the piston **103** may be programmed into the electronic control module **900,** such that the plunger **113** may be retracted automatically or may be initiated by a technician or physician by entering a command via the user interface **124.** In other examples, the technician or physician may manually control the piston **103** and the plunger **113** via the user interface **124.** With reference to **FIGS. 8A-8C,** the plunger **113** may be moved from a starting position $x_0$ to a final ending position $x_f$. The starting position $x_0$ may represent a position within the syringe **132** located distally relative to the final ending position $x_f$. For example, the starting position $x_0$ may be a position within the syringe **132** corresponding to the 100 mL initial volume of fluid drawn into the syringe **132,** and the final ending position $x_f$ may be a position of the syringe **132** that represents 120 mL, thus creating a displacement of 20 mL of the plunger **113.** Other final ending positions $x_f$ may be used depending on the vacuum pressure required to remove the amount of small air bubbles desired, according to the methods herein.

In some examples, the at least partial vacuum placed on the at least one fluid reservoir may be automated. In some examples, the electronic control module **900** may be configured to automatically generate the at least partial vacuum by actuating the piston **103** and drawing in the proximal direction after the initial volume of fluid is drawn into the syringe **132** and the valve **136** is closed in steps **802-804.** In other examples, the electronic control module **900** may be configured to automatically generate the at least partial vacuum by actuating the piston **103** upon detection of gas bubbles **61** remaining in the volume of fluid drawn into the syringe at step **802,** for example after a bulk air purge process. In other examples, the electronic control module **900** may be configured to generate the at least partial vacuum by actuating the piston **103** upon manual entry of a command into the user interface **124** by the technician or physician. In certain embodiments, the control module **900** may be configured to determine the amount of adhered gas bubbles on the interior surfaces of the fluid reservoir, and utilize an algorithm or look-up table based on the amount of adhered gas bubbles to determine and implement the minimum at least partial vacuum necessary to effect removal of the adhered gas bubbles using the methods described herein.

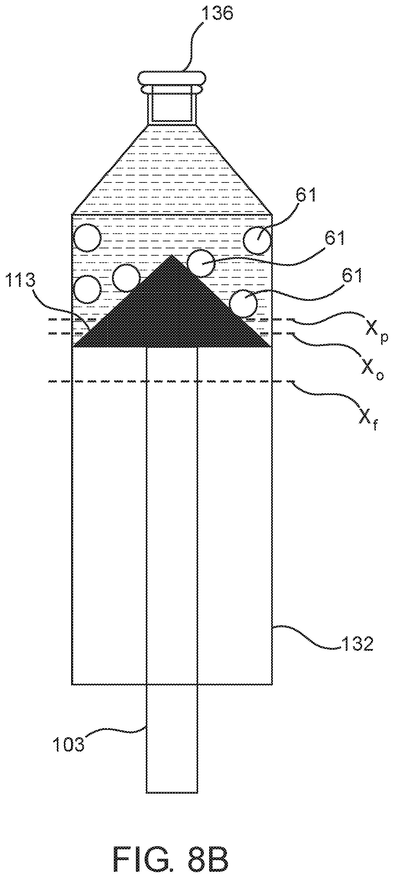

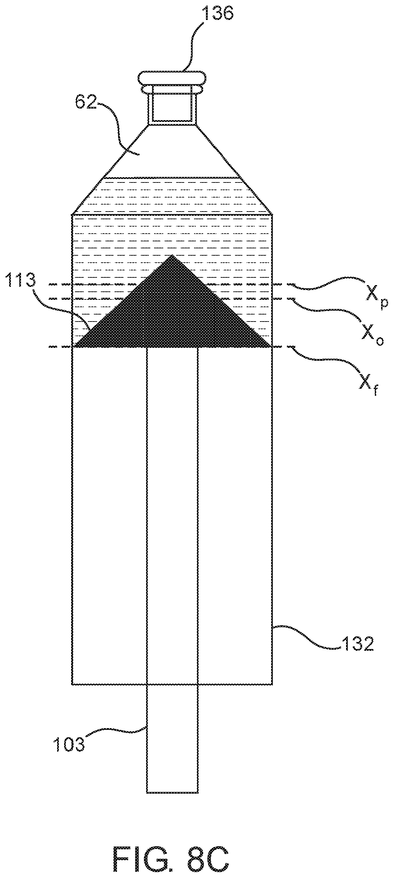

With continued reference to step **806,** the gas bubbles **61** may be dislodged from the interior surface of the fluid reservoir, for example syringe **132,** due to the creation of the at least partial vacuum. With reference to **FIG. 8B,** as a consequence of the change in pressure ($\Delta$P) generated within the syringe **132,** the pressure within the fluid and gas contents of the closed system, including the pressure within each bubble **61,** is reduced which causes each bubble **61** to expand in volume and increase in overall surface area and buoyancy. Moreover, the proportion of the surface area of each bubble **61** in contact with the at least one interior surface of the syringe **132** may be reduced due to the volumetric expansion of each bubble **61.** Referring now to **FIG. 8C,** the increased buoyancy and reduced contact area with the at least one interior surface of the syringe **132** of each bubble may overcome the adhesion forces between the bubbles **61** and the at least one interior surface of the syringe **132,** inducing the bubbles **61** to dislodge from the at least one interior surface of the syringe **132** and float to the distal end or highest point of the syringe **132.** As the bubbles **61** rise to the distal end or highest point in the syringe **132,** the bubbles **61** may develop an affinity to coalesce with one another and form a coalesced bubble **62** of larger volume. The coalesced bubble **62** may have a reduced effective surface area in comparison to the combined effective surface areas of the individual bubbles **61.** The reduction in effective surface area may in turn minimize the total surface tension and surface energy associated with coalesced bubble **62.**

It is noted that while **FIG. 8B** shows the expansion of the bubbles **61** and **FIG. 8C** shows the coalescence of the bubbles **61** into the coalesced bubble **62,** the expansion and coalescence of the bubbles **61** may in reality not be discrete steps, but may rather occur gradually and at least partially simultaneously as the at least partial vacuum is generated within the syringe **132.** According to certain embodiments, if sufficient vacuum, as determined by the ∆P, is generated within the syringe **132,** the reduced pressure may also draw dissolved gases out of the fluid which may coalesce with the coalesced bubble **62.**

With continued reference to **FIGS. 7-8C,** at optional step **808,** dislodging the gas bubbles **61** from the at least one interior surface of the fluid reservoir may be assisted by applying at least one force to the fluid injector system **100.** In one example, this force may encompass a single or multiple impacts to at least one portion of the fluid injector system at or near the final ending position $x_f$ while the fluid in the at least one fluid reservoir of an injector system **100** is under an at least partial vacuum. The single or multiple impacts may be made to the syringe **132** via actuation of the piston **103** or by a separate mechanism that creates one or more impacts to the fluid injector system **100.** The single impact may also be made to any portion of the fluid path set in fluid communication with the fluid filled reservoir, such as the manifold **148.** In another example, the at least one force may include at least one vibration, performed at a specific or randomized frequency and amplitude, of the fluid injector system **100.** The at least one vibration may be created by a vibrational actuator **68** that produces an oscillating force across a single axis, such as a linear resonant actuator or an eccentric rotating mass motor. The vibrational actuator **68** may be located along at least one portion of the fluid injector system **100,** such as at a portion of the outer surface of the fluid filled reservoir or a portion of the outer surface of the any portion of the fluid path set, such as the manifold **148.** In another example, the vibrational actuator **68** may be located on or within the piston **103.** The vibrational actuator, wherever located, ultimately causes vibration of the surfaces of the fluid reservoir, the surfaces of at least a portion of the fluid path set in fluid communication with the fluid reservoir, and/or the fluid within the syringe **132.** In other examples, the fluid reservoir may be vibrated by pulsatile movements of the piston **103.** In this example, the electronic control module **900** may instruct the piston **103** to pulsate, e.g., rapidly move by small displacements in the distal and proximal directions, thereby moving the plunger **113,** which in turn may produce vibrations of the fluid within the syringe **132.** As noted above, step **808** is optional and may be omitted in some examples of the present disclosure. According to these embodiments, the application of the at least one force to the fluid injector system **100** may dislodge one or more of the gas bubbles **61,** for example by decreasing the contact surface area between the gas bubble **61** and the interior surface and allowing the buoyancy of the gas bubble **61** to overcome the surface tension adhesive forces of the reduced surface area of contact.

With continued reference to **FIG. 7,** at optional step **810,** the fluid reservoir may be pressurized to ensure that the coalesced bubble **62** remains coalesced and is forced to a distal-most region of the syringe **132.** Further, under the increased pressure, the coalesced bubble **62** is immediately moved down the fluid path when the valve **136** is moved to the open position (at step **812),** such that the coalesced bubble **62** is transferred to the bulk fluid reservoir or purged from the system as the valve **136** is opened. Pressurization of the coalesced bubble **62** may be achieved by moving the plunger **113** distally in the syringe **132** via the piston **103** while the fluid injector system **100** is in the closed position. For example according to a non-limiting embodiment, if during step **806** the plunger **113** is proximally displaced 20 mL (e.g. from the starting position $x_0$ corresponding to 100 mL to the final ending position $x_f$ corresponding to 120 mL), the plunger **113** may then be moved distally 21 mL, to a position $x_p$ corresponding to 99 mL within the syringe **132,** to pressurize the coalesced bubble **62.** Pressurization of coalesced bubble **62** may be performed automatically by the electronic control module **900.** For example, the electronic control module **900** may be programmed or configured to wait a predetermined amount of time after generating the at least partial vacuum at step **806** to ensure that the individual bubbles **61** have had sufficient time to coalesce into the coalesced bubble **62.** After the predetermined amount of time has elapsed, the electronic control module **900** may automatically instruct the piston **103** to move distally to position $x_p$ to pressurize the coalesced bubble **62.** In other examples, pressurization of the coalesced bubble **62** may be performed manually via the technician or physician entering a command into the user interface **124.**

With continued reference to **FIG. 7,** at step **812,** the fluid injector system **100** is transformed into an open system, such that the at least one fluid reservoir is in fluid communication with atmospheric pressure. In some examples, the electronic control device **900** may be configured to transform the fluid injector system **100** from a closed system to an open system opening, for example by rotating or otherwise adjusting, the valve **136** to place the at least one fluid reservoir, e.g. the at least one syringe **132,** in fluid communication with atmospheric pressure, for example either in fluid communication with the fluid path to the bulk fluid containers or in fluid communication with the tubing set that will be connected to the patient after gas removal. In other examples, valve **136** may be manually rotated by a technician or a physician to place the at least one syringe **132** in fluid communication with atmospheric pressure.

With continued reference to **FIG. 7,** at step **814,** the coalesced bubble **62** is purged from the at least one fluid reservoir once the fluid injector system **100** is transformed into an open system. In some examples, the electronic control module **900** may actuate the piston **103** to move the plunger **113** distally in the syringe **123** such that the coalesced bubble **61** is expelled from the fluid path set downstream of the syringe **132,** such as the manifold **148** and/or the one or more fluid outlet lines **152** or into the bulk fluid reservoir. In some examples, purging the coalesced bubble **62** from the syringe **132** may be achieved by distally advancing the plunger **113,** via the piston **103,** a predetermined distance according to an estimated volume of the coalesced bubble **62** within the syringe **132** and the volume of the fluid path that the bubble travels through while being purged, such as, for example, 20 mL,. Alternatively, if optional step **810** is performed to pressurize the coalesced bubble **62,** the total piston movement distance may be reduced. In some examples, coalescing any adhered bubbles **61** and purging the coalesced bubble **62** may be performed automatically as part of a filling, priming, or injection procedure. In some examples, the electronic control module **900** may instruct the piston **103** to purge the coalesced bubble **62** prior to initiating a diagnostic injection protocol. In other examples, purging of the coalesced bubble **62** may be performed manually via the technician or physician entering a command into the user interface **124.**

With continued reference to **FIG. 7,** at step **816,** the fluid reservoir is filled to a total volume (i.e. 100% volume) of fluid prescribed by the diagnostic injection protocol to account for the volume lost during purging of the coalesced bubble **62.** In some examples, the filling may be achieved by proximally retracting plunger **113,** via piston **103,** a predetermined distance, corresponding to a predetermined volume, within syringe **132.** At this stage, the fluid reservoir may be filled with the volume of fluid necessary to complete the diagnostic injection protocol and substantially all of the visible gas has been purged. As such, the fluid reservoir is prepared for fluid injector system **100** for the diagnostic injection protocol.

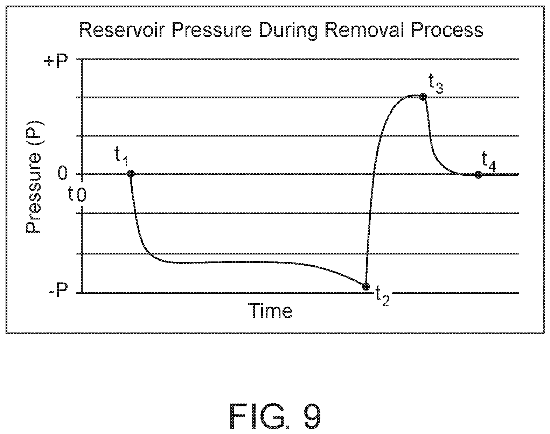

A graphical representation of the pressure within the fluid reservoir as a function of time during performance of one embodiment of the method **800** is shown in **FIG. 9.** Pressure (P) is shown on the y-axis of **FIG. 9,** with (+P) indicating a positive pressure relative to an atmospheric pressure, e.g. atmospheric pressure within the fluid source **120,** and (-P) indicating a reduction in pressure, or vacuum pressure, relative to an atmospheric pressure, e.g. atmospheric pressure within the fluid source **120.** Time (t) is shown on the x-axis of **FIG. 9** and increases from an initial time $t_0$ to a final time $t_f$. At time $t_0$, the pressure (P) within the fluid reservoir is approximately equal to the atmospheric pressure of a component, e.g. the fluid source **120,** to which the fluid reservoir is in fluid communication. At some time between time $t_0$ and $t_1$, the initial volume of fluid is drawn into the fluid reservoir and the injector system is transformed into a closed system, as described herein with reference to steps **802-804** of the method **800.** At time $t_1$, an at least partial vacuum is placed on the fluid reservoir, as described herein with reference to step **806.** As such, the pressure (P) within the fluid reservoir decreases from time $t_1$ to time $t_2$ until a maximum negative pressure (i.e. a maximum vacuum) is reached at time $t_2$. At time $t_2$, the vacuum is relieved and the fluid reservoir is pressurized as describe herein with reference to step **810.** At time $t_3$, once the desired pressurization of the fluid reservoir is attained, the fluid reservoir is at a maximum positive pressure. At time $t_3$, the fluid injector system is transformed into an open system, as described herein with reference to step **812,** causing the pressure within the fluid reservoir to be relieved. The pressure within the fluid reservoir decreases from the maximum pressure at time $t_3$ to atmospheric pressure at time $t_4$, as the coalesced bubble is purged from the fluid reservoir as described herein with reference to step **814.** After time $t_4$, filling of the fluid reservoir according to step **816** may be performed, followed by performance of the injection protocol. It is noted that the graphical representation illustrated in **FIG. 9** is merely exemplary of certain embodiments of the method **800** and may not be shown to scale.

In some examples of the present disclosure, the method **800,** as performed by the fluid injector system **100,** may be implemented by a computer program product. The computer program product may include at least one non-transitory computer-readable medium having one or more instructions executable by at least one processor to cause the at least one processor to execute all or part of the method **800.** In some examples or aspects, the at least one non-transitory computer-readable medium and the at least one processor may include or correspond to the memory **908** and processor **904,** respectively, as described above with reference to **FIG. 4.**

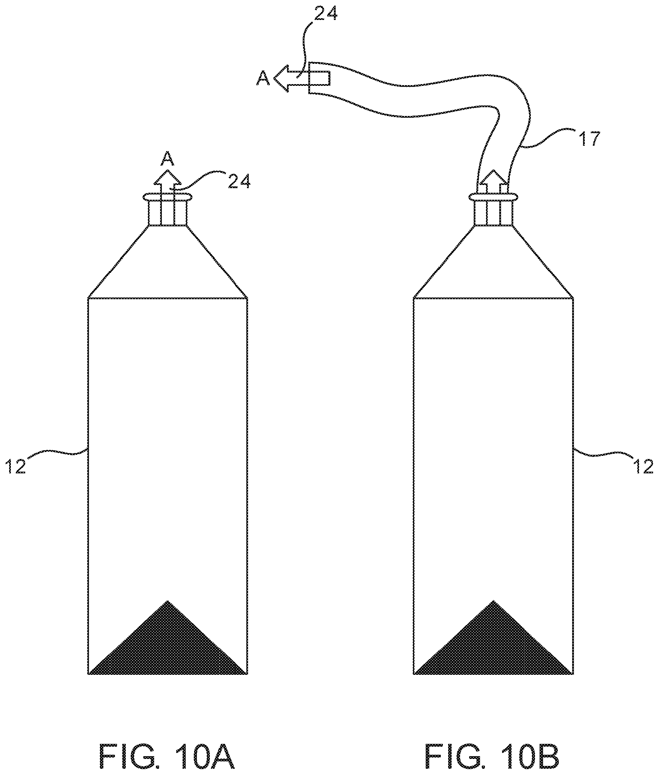

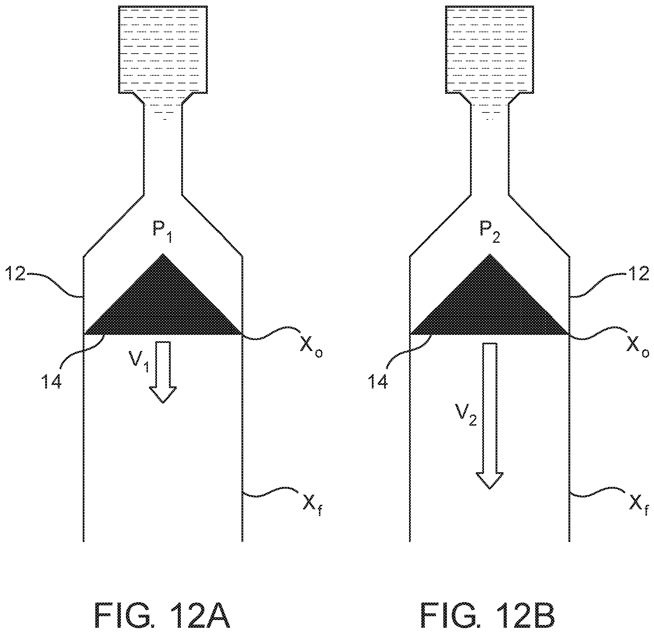

In other examples of the present disclosure, a method **850** of gas removal may be utilized in an open fluid injector system **100,** meaning that the at least one fluid reservoir of the fluid injector system **100** remains in fluid communication with atmospheric pressure throughout the steps of the method **850.** For simplicity, embodiments of the method **850** may be described primarily with respect to the example of the fluid injector system **100** described in connection with **FIGS. 5, 10A,B,** and **12A,B** although the process may be utilized by other open fluid injection systems. **FIGS. 10A,B** and **12A,B** show manners in which the fluid reservoirs, e.g. the syringes **12** of the fluid injector system **100** of **FIG. 5,** may be in fluid communication with atmospheric pressure **A,** such as atmospheric pressure **A** of the fluid source. In **FIG. 10A,** an outlet **24** of the syringe **12** is in direct fluid communication with atmospheric pressure **A.** In **FIG. 12B,** the outlet **24** of the syringe **12** is in fluid communication with atmospheric pressure **A** via the fluid path set **17.** The method **850** may also be used with examples of the fluid injector system **100** of **FIGS. 1-3B,** provided that the valves 136 thereof are positioned to maintain fluid communication with atmospheric pressure throughout the steps **858-876** (described herein) of the method **850.**

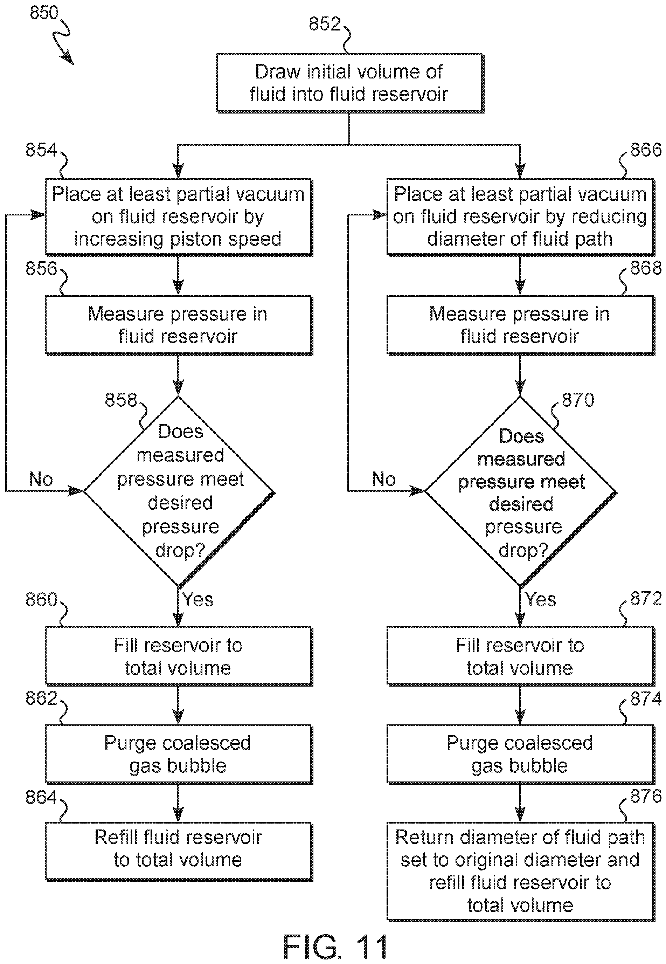

With reference now to **FIG. 11-12B,** at step **852** of the method **850,** an initial volume of fluid is drawn into at least one fluid reservoir, e.g. at least one syringe **12,** of the fluid injector system **100,** similar to the manner in which fluid is drawn into the syringe **132** at step **802** of the method **800** for a closed or closeable system. Particularly, the initial volume of fluid may be drawn into the fluid reservoir by retracting the piston **103** (not shown in **FIG. 5)** connected to the plunger **14** in substantially the same manner described in step **802** of the method **800** for a closed or closeable system. The initial volume of fluid drawn into the fluid reservoir may be a portion of the desired final fill volume, for example, approximately 70% of a desired fill volume of the fluid reservoir. The desired fill volume may correspond to at least the prescribed volume of fluid to be injected into the patient according to a predetermined diagnostic injection protocol. In examples in which the fluid reservoir is the syringe **12,** the location of the plunger **14** within the syringe **12,** once the initial volume has been drawn into the syringe **12,** may correspond to the starting position $x_0$.

With continued reference to **FIG. 11-12B,** at steps **854-858,** an at least partial vacuum may be placed on the fluid reservoir, e.g. the at least one syringe **12.** The at least partial vacuum may be created by generating a pressure drop across the open fluid injector system **100** by retracting the piston and plunger at a rate greater than the vacuum can be replaced. In a general form, the pressure drop may be calculated using equation (1):

$$\Delta P = A\left(\frac{\eta Q L}{d^4}\right) \tag{1}$$

wherein $\Delta P$ is the pressure drop across the open fluid injector system **100** (which may be measured in any appropriate pressure unit, such as in pounds per square inch ("psi"), atmospheres (atm), or Pascals ("Pa")), $\eta$ is a viscosity of at least one fluid being delivered from the fluid injector system to the patient (which may be measured in any appropriate viscosity unit, such as in centipoise ("CP"), A is a geometric constant of the fluid path, Q is the volumetric flow rate of at least one fluid being pulled into the fluid injector system through the outlet (which may be measured in any appropriate volumetric flow rate unit, such as in ("mL/s")), L is the length of the fluid path, and d represents a diameter of the fluid path. Parameters A, L and d will vary depending upon the fluid path or the portion of the fluid injector system being measured. Yet, one skilled in the art would appreciate that other factors, such as fluid path geometry or orientation, may be able to be manipulated in order to obtain a desired pressure drop. The desired pressure drop $\Delta P$ represents the at least partial vacuum needed to overcome the atmospheric pressure **A** that the open fluid injector system is exposed to via the outlet **24,** and to dislodge and coalesce any gas bubbles present in the initial volume of fluid drawn into the fluid reservoir.

As may be appreciated from the above pressure drop equation, the desired pressure drop $\Delta P$ for a given fluid may be obtained by altering either the flow rate Q, the diameter d at a portion of the fluid path, the length L of the fluid path, or a combination thereof. In one example of the present disclosure, described in steps **854-864,** the pressure drop $\Delta P$, and hence the at least partial vacuum, may be generated through an incremental change in flow rate Q of the at least one fluid. In particular, after the initial volume of fluid has been drawn into the fluid reservoir at step **852,** or concurrently with drawing the initial volume of fluid into the fluid reservoir at step **852,** the at least partial vacuum is placed on the fluid reservoir at step **854.** In examples in which the fluid reservoir is the syringe **12,** the at least partial vacuum may be created by the electronic control module **900** actuating the piston **103** to move the plunger **14** proximally from the starting position $x_0$ towards the final ending position $x_f$. The electronic control module **900** may first actuate the piston **103** at a predetermined initial speed, which may be later adjusted, if necessary, to achieve the desired pressure drop $\Delta P$. In particular, at step **856,** the actual pressure within the fluid reservoir may be intermittently or continuously monitored as the piston **103** moves the plunger **14** between the starting position $x_0$, and the final ending position $x_f$. Measurement of the pressure may be obtained, for example, via monitoring of the motor current of the motor driving the piston **103,** monitoring the position of the plunger within the syringe, optical measurement of reservoir deformation, or by a pressure sensor in communication with the fluid path set **17** or the piston **103.** At step **858,** the electronic control module **900** determines whether the measured pressure drop meets or exceeds the desired pressure drop $\Delta P$, and, consequently, the at least partial vacuum necessary to dislodge and coalesce the gas bubbles present in the initial volume of fluid drawn into fluid reservoir. The process by which the bubbles dislodge from at least one interior surface of the fluid reservoir (e.g. the sidewall of the syringe **12** and/or a surface of the plunger **14)** and coalesce into a coalesced bubble in the fluid reservoir is substantially the same as the process described with reference to **FIGS. 8A-8C.**

Referring again to step **858,** if the electronic control module **900** determines that the measured pressure in the fluid reservoir is below the desired pressure drop $\Delta P$, the electronic control module **900** may return to step **854** and increase the speed of the piston **103.** The pressure in the fluid reservoir is then re-measured at step **856** to determine if the pressure meets or exceeds the desired pressure drop $\Delta P$. Steps **854** and **856** may be repeated as many times as necessary to achieve the desired pressure drop $\Delta P$, with the speed of the piston **103** being incrementally increased at each iteration. According to various embodiments, repetition of steps **854** and **856** may result in additional impact forces to the syringe that may assist in dislodging the gas bubbles from the interior surfaces. In some examples, as shown in **FIGS. 12A** and **12B,** the electronic control module **900** may be programmed to automatically increase the retraction speed of the piston **103.** For example, electronic control module **900** may initially retract the piston **103** at a first speed **V1** of 1 mL/s, as shown in **FIG. 12A.** The electronic control module **900** may then increase the retraction speed of the piston **103** to a second speed **V2** of 2 mL/s, as shown in **FIG. 12B,** if the desired pressure drop $\Delta P$ is not detected at step **856.** A first vacuum pressure **P1** in the fluid reservoir generated by the first speed **V1** may be less than a second vacuum pressure **P2** generated by the second speed **V2.** The speed of retraction of the piston **103** may be continually increased in any predetermined or dynamically calculated increment until the desired pressure drop $\Delta P$ has been attained.

If at step **858,** the electronic control module **900** determines that the measured pressure in the fluid reservoir meets or exceeds the desired pressure drop $\Delta P$, the electronic control unit **900** may cease altering the speed of the piston **103.** In some examples, if the measured pressure in fluid reservoir exceeds the desired pressure drop $\Delta P$ by a predetermined value, step **856** may be repeated except that the retraction speed of the piston **103** may be reduced to reduce the pressure drop.

With continued reference to **FIG. 11,** at step **860,** the fluid reservoir may optionally be filled to the total volume prescribed by the diagnostic injection protocol. In some examples, the electronic control module **900** may actuate the piston **103** at a constant speed until the plunger **14** has reached the final ending position $x_f$ within the syringe **12.** The final ending position $x_f$ may correspond to a total volume (i.e. 100% volume) of the fluid required by the diagnostic injection protocol. However, at this stage, some of the volume of the syringe **12** between the final ending position $x_f$ and the outlet **24** may be occupied by the coalesced air bubble. In other examples, the final ending position $x_f$ may correspond to a volume greater than the volume of fluid required by the diagnostic injection protocol, such that the total volume of fluid contained within the fluid reservoir after expulsion of the gas meets or exceeds the volume of fluid required by the diagnostic injection protocol.

In some examples, a flow control device, such as a flow diverter, may be positioned in proximity to the outlet **24.** The flow diverter may direct the flow of fluid into the syringe **12** so as to avoid breaking or dividing the coalesced bubble formed in the syringe **12.** In particular, the flow diverter may induce a Coandă effect such that incoming fluid flows around an outer surface of the coalesced bubble, thereby mitigating forces of fluid flow that could otherwise overcome the surface tension of the coalesced bubble and cause the coalesced bubble to fracture into multiple smaller bubbles.

With continued reference to **FIG. 11,** at step **862,** the coalesced bubble is purged from the at least one fluid reservoir in the same manner described in step **814** of the method **800** of **FIG. 7.** At step **864,** the at least one fluid reservoir is filled to the total volume (i.e. 100% volume) of fluid prescribed by the diagnostic injection protocol in the same manner described in step **816** of the method **800** of **FIG. 7.**

As noted above, steps **854-864** correspond to examples of the present disclosure in which the desired pressure drop $\Delta P$ is achieved by altering the flow rate Q of the fluid. In other examples, the desired pressure drop $\Delta P$ is achieved by altering the diameter d of at least a portion of the fluid path set **17.** In such examples, steps **866-876** of the method **850** may be performed in place of steps **854-864.**

In particular, after the initial volume of fluid has been drawn into the fluid reservoir at step **852,** or concurrently with drawing the initial volume of fluid into the fluid reservoir at step **852,** the diameter d of at least a portion of the fluid path set **17** may be decreased at step **866** to increase the pressure drop $\Delta P$ across the decreased diameter as the fluid is drawn through the fluid path set **17.** In particular, the decreased diameter creates a restriction within the fluid path set **17** such that fluid exits the decreased diameter at a reduced pressure. In examples in which the fluid reservoir is the syringe **12,** the electronic control module **900** may actuate the piston **103** to move the plunger **14** at a constant speed or at a varying speed (as described herein) from the starting position $x_0$ towards the final ending position $x_f$. As the fluid is drawn through the restriction created by the decreased diameter of the portion of the fluid path set **17,** the pressure in the fluid reservoir decreases.

With continued reference to **FIG. 11,** at step **868,** the actual pressure within the fluid reservoir may be intermittently or continuously monitored as the piston **103** moves the plunger **14** between the starting position $x_0$, and the final ending position $x_f$. Measurement of the pressure may be obtained via monitoring of the motor current of the motor driving the piston **103,** monitoring the position of the plunger within the syringe **12,** optical measurement of reservoir deformation, or by a pressure sensor in communication with the fluid path set **17** or the piston **103.** At step **870,** the electronic control module **900** determines whether the measured pressure drop meets or exceeds the desired pressure drop $\Delta P$, and, consequently, the at least partial vacuum necessary to dislodge and coalesce the gas bubbles present in the initial volume of fluid drawn into fluid reservoir. The process by which the bubbles dislodge from at least one interior surface of the fluid reservoir (e.g. the sidewall of the syringe **12** and/or a surface of the plunger **14**) and coalesce into a coalesced bubble in the fluid reservoir is substantially the same as the process described with reference to **FIGS. 8A-8C.**

Referring again to step **870,** if the electronic control module **900** determines that the measured pressure in the fluid reservoir is below the desired pressure drop $\Delta P$, the electronic control module **900** may return to step **866** and further decrease the diameter d of the portion of the fluid path set **17,** while the speed of the piston **103** remains constant.

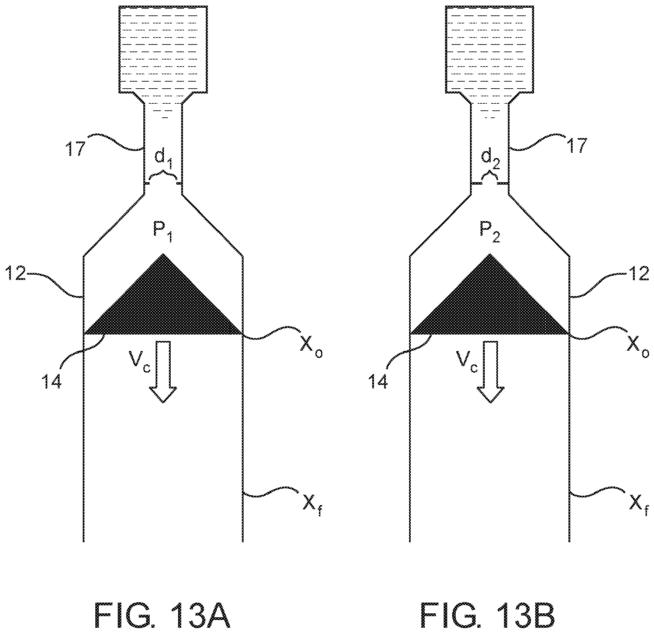

The pressure in the fluid reservoir is then re-measured at step **868** to determine if the pressure meets or exceeds the desired pressure drop ΔP. Steps **866** and **868** may be repeated as many times as necessary to achieve the desired pressure drop ΔP, with the diameter d of the portion of the fluid path set **17** being incrementally decreased at each iteration. In some examples, if the measured pressure in fluid reservoir exceeds the desired pressure drop ΔP by a predetermined value, step **866** may be repeated except that the diameter d may be increased to reduce the pressure drop. According to various embodiments, repetition of steps **866** and **868** may result in additional impact forces to the syringe that may assist in dislodging the gas bubbles from the interior surfaces. In some examples, as shown in **FIGS. 13A** and **13B,** the electronic control module **900** may be programmed to automatically decrease the diameter d of the portion of the fluid path set **17.**

In some examples, the electronic control module **900** may decrease the diameter d of the portion of the fluid path set **17** by closing a gate valve or pinch valve disposed in the fluid path set **17.** In other examples, the electronic control module **900** may decrease the diameter d by modulating the opening and closing of a valve disposed in the fluid path set **17.** In still other examples, the electronic control module **900** may decrease the diameter d by actuating a portion of the fluid path set **17** configured to change in size in response to temperature, voltage, current, magnetism, etc. The diameter may initially have a first diameter **d1,** as shown in **FIG. 13A,** while the plunger **14** is retracted at a constant velocity $V_c$. The electronic control module **900** may then decrease the diameter to a second diameter **d2,** as shown in **FIG. 13B,** if the desired pressure drop ΔP is not detected at step **856.** The plunger **14** is maintained at the constant retraction velocity $V_c$. A first vacuum pressure **P1** in the fluid reservoir resulting from the first diameter **d1** may be less than a second vacuum pressure **P2** resulting from the second diameter **d2.** The diameter of the portion of the fluid path set **17** may be continually decreased and/or increased in any desired increment until the desired pressure drop ΔP has been attained.

If at step **858,** the electronic control module **900** determines that the measure pressure in the fluid reservoir meets or exceeds the desired pressure drop ΔP, the electronic control unit **900** may cease altering the diameter of the fluid path set **17.**

With continued reference to **FIG. 11,** at step **872,** the fluid reservoir may optionally be filled to the total volume prescribed by the diagnostic injection protocol in the same manner as step **860.** At step **874,** the coalesced bubble is purged from the at least one fluid reservoir in the same manner described in step **862.** At step **876,** the diameter of the portion of the fluid path set **17** is returned to its original diameter, and the at least one fluid reservoir is filled to the total volume (i.e. 100% volume) of fluid prescribed by the diagnostic injection protocol in the same manner described in step **864.**

Having generally described the steps of the method **850,** particular processes for adjusting the pressure drop ΔP in the fluid reservoir will now be described in greater detail. As described herein, the at least one fluid for filling the at least one fluid reservoir may be a saline solution, a contrast agent, or any other medical fluid that may be needed for a medical or diagnostic injection protocol. These fluids may have different viscosities, with more viscous fluids, such as the contrast agent, having characteristics such as concentration, more resistant to fluid flow than less viscous fluids, such as the saline solution. Further, different contrast agents may have different viscosities due to the concentrations of dissolved contrast and different saline flushing agents may similarly have different viscosities based on solution concentrations. Additionally, temperature of the fluid may have an effect on the viscosity of the fluid and the surface adhesion properties. As such, the fluid flow rate necessary to generate a given pressure drop may vary, at least in part, depending on the viscosity of the fluid. Additionally, the vacuum pressure necessary to cause the gas bubbles to dislodge and coalesce may vary, at least in part, based on the viscosity and other molecular and/or physical properties of the fluid. In particular, the molecular and/or physical properties of the given fluid may dictate, at least in part, the size increase of the bubbles necessary to overcome the surface adhesion of the bubbles to the at least one interior surface of the fluid reservoir. As such, at steps **854** and **866** of the method **850,** the electronic control module **900** may determine or estimate the necessary vacuum pressure, i.e. the pressure drop ΔP, to overcome the surface adhesion of the bubbles based on known properties of the fluid and/or gas drawn into the fluid reservoir. For example, the electronic control module **900** may interpolate the necessary vacuum pressure from an empirical data set relating the initial and final bubble size to the necessary vacuum necessary to overcome the bubble adhesion force. In a similar manner, at step **866,** the electronic control module **900** may estimate the necessary diameter of the portion of the fluid path set **17** based on known properties of the fluid and/or gas drawn into the fluid reservoir. In other examples, known properties of the fluid and/or gas may include at surface tension of the fluid relative to the interior surface, surface tension of the gas relative to the interior surface, surface texture of the at least one interior surface of the fluid reservoir, and buoyancy of the gas bubbles in the specific fluid.

In some examples of the present disclosure, the method **850,** as performed by the fluid injector system **100,** may be implemented by a computer program product. The computer program product may include at least one non-transitory computer-readable medium having one or more instructions executable by at least one processor to cause the at least one processor to execute all or part of the method **850.** In some examples or aspects, the at least one non-transitory computer-readable medium and the at least one processor may include or correspond to the memory **908** and processor **904,** respectively, as described above with reference to **FIG. 4.**

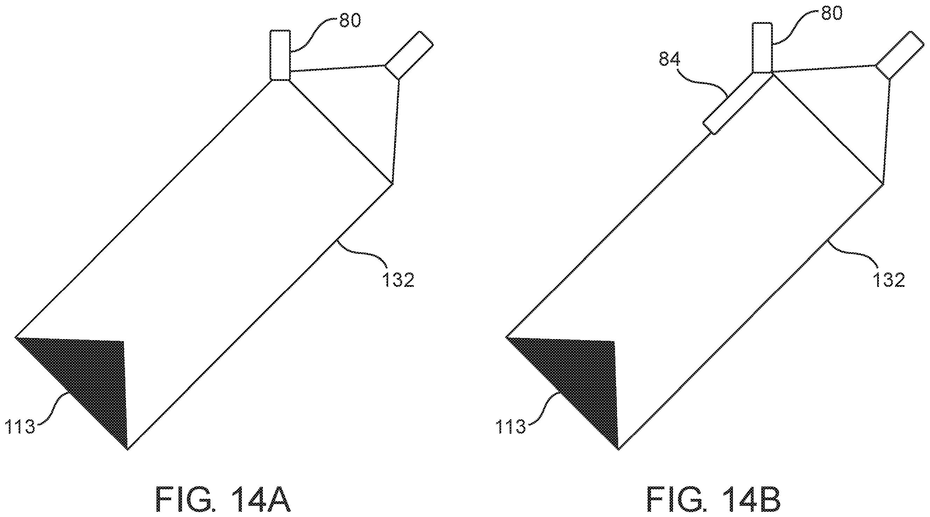

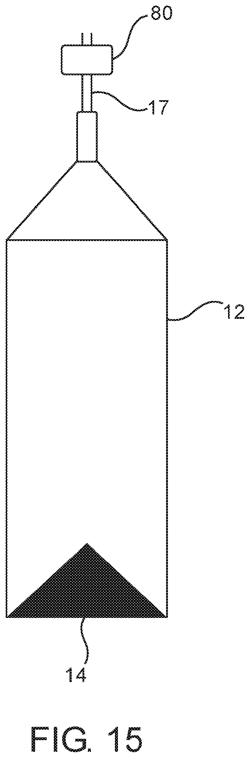

Referring now to **FIGS. 14A-15,** in some examples of the present disclosure, expulsion of the coalesced gas bubble **62** (see **FIGS. 8A-8C)** at step **814, 862,** or **874** may be facilitated by at least one gas collection chamber **80** located within or attached to the fluid reservoir. Upon coalescing at the highest point of the fluid reservoir, e.g. the syringe **132,** the at least one coalesced bubble **62** may accumulate in the gas collection chamber **80.** In one example, illustrated in **FIG. 14B,** the gas collection chamber **80** may be in fluid communication with a channel **84** to guide the gas to the gas collection chamber **80.** In another example, referring to **FIG. 15,** the gas collection chamber **80** may be located along at least one portion of the fluid path set **17** connected to the fluid reservoir, e.g. the syringe **12.** The at least one coalesced air bubble may be expelled out of the fluid reservoir, e.g. the syringe **12,** travel along the fluid path set **17,** and accumulate within the gas collection chamber **80.** Alternatively, the gas collection chamber **80** may be configured so that the coalesced bubble may be in a position where expulsion during a fluid injection protocol is not possible and the coalesced bubble is retained in the gas collection chamber **80.** In still other embodiments, an adsorptive material may be located in the gas collection chamber **80** to react with or adsorb the gas, thereby removing the gas from the injection solution.

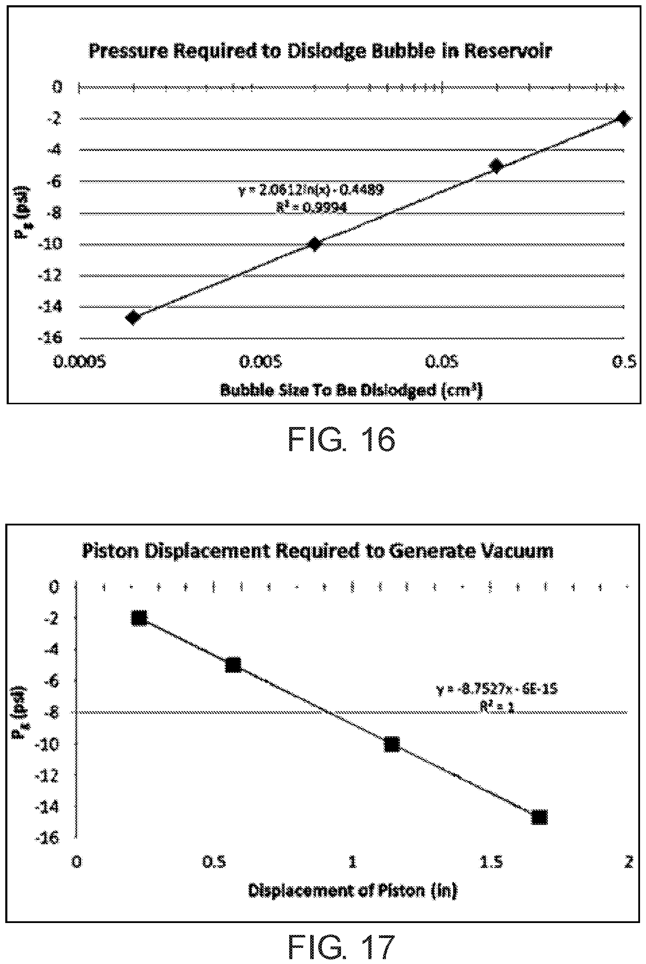

Referring now to **FIGS. 16** and **17,** in some examples of the methods **800** and **850,** the at least partial vacuum generated at steps **806, 854,** and **866** may be determined by implementation of an algorithm and/or a look-up table. In particular, the electronic control module **900** may be configured to determine the value of the at least partial vacuum for a given fluid based on an algorithm and/or a look-up table relating the size of the bubble to be dislodged to a necessary vacuum pressure. **FIG. 16** shows a graphical representation of an equation relating bubble size in cubic centimeters (cm$^3$) to necessary gauge pressure ($P_g$) for a given fluid contained within the fluid reservoir. Bubble size (cm$^3$) increases along the x axis, while gauge pressure ($P_g$) decreases going down the y-axis. The electronic control module **900** may execute an algorithm incorporating the equation to determine the gauge pressure necessary to dislodge the bubbles from the at least one interior surface of the fluid reservoir. In some examples, the algorithm executed by the electronic control module **900** may further include an equation and/or a look-up table for determine the retraction of the piston **103** necessary to attain the determined vacuum. **FIG. 17** shows a graphical representation of an equation relating displacement or retraction of the piston **103** in inches (in) to gauge pressure ($P_g$) for a given fluid reservoir. Piston displacement or retraction (in) increases along the x axis, while gauge pressure ($P_g$) decreases going down the y-axis. The equation for determining displacement of the piston **103** to achieve the necessary gauge pressure may be a function of the physical properties of the fluid reservoir, such as an internal cross sectional area of the fluid reservoir perpendicular to the retraction direction of the piston **103.**

While several examples of fluid delivery systems, computer program products, and methods of use thereof are shown in the accompanying drawings and described hereinabove in detail, other examples will be apparent to, and readily made by, those skilled in the art. For example, it is to be understood that this disclosure contemplates that, to the extent possible, one or more features of any example can be combined with one or more features of any other example. Accordingly, the foregoing description is intended to be illustrative rather than restrictive.

**Claims**

**1.** A fluid injector system (100), comprising:

at least one fluid reservoir having at least one interior surface and defining an internal volume;
at least one actuator configured to change the internal volume of the at least one fluid reservoir (132); and
at least one processor (904) programmed or configured to:

drive the actuator to at least partially fill the at least one fluid reservoir with a fluid from a fluid source (120);
drive the actuator to generate at least a partial vacuum within the internal volume to dislodge one or more gas bubbles (61) adhered to the at least one interior surface and to cause the one or more gas bubbles (61) to coalesce into a single coalesced bubble (62); and
drive the actuator to expel the single coalesced bubble (62) from an outlet of the at least one fluid reservoir;
**characterized in that**
the at least one processor (904) is further programmed or configured to determine and initiate the necessary actuator displacement necessary to achieve the partial vacuum to dislodge the one or more gas bubbles (61) from the at least one interior surface based on at least one of a surface tension of the fluid, a surface tension of the gas, a surface texture of the at least one interior surface, interfacial surface energy between the at least one interior surface and the gas, a weight of the fluid above the one or more gas bubbles (61) in the fluid reservoir, and a buoyancy of the one or more gas bubbles (61) in the fluid.

**2.** The fluid injector system (100) of claim 1, wherein the at least one processor (904) is further programmed or configured to, prior to driving the actuator to generate at least the partial vacuum within the internal volume, close the outlet of the at least one fluid reservoir to fluidly isolate the internal volume.

3. The fluid injector system (100) of claim 2, wherein the at least one processor (904) is further programmed or configured to, after closing the outlet of the at least one fluid reservoirand after generating the partial vacuum within the internal volume, drive the actuator to pressurize the single coalesced bubble (62).

4. The fluid injector system (100) of claim 3, wherein the at least one processor (904) is further programmed or configured to, prior to driving the actuator to expel the single coalesced bubble (62) from an outlet of the at least one fluid reservoir, open the outlet of the at least one fluid reservoir to provide fluid communication between the internal volume and one of the fluid source (120) and a fluid outlet pathway (152).

5. The fluid injector system (100) of claim 1, wherein the at least one fluid reservoir comprises a syringe (12, 132), and wherein the actuator comprises a piston (103) configured to move reciprocally to change the internal volume of the syringe (12, 132).

6. The fluid injector system (100) of claim 5, wherein the syringe (12, 132) further comprises a plunger (14) slideable within the syringe (12, 132), wherein the at least one interior surface further comprises a surface of the plunger (14), and wherein the piston (103) is releasably connected to the plunger (14) and is configured to reciprocally move the plunger (14) within the syringe (12, 132).

7. The fluid injector system (100) of claim 5, wherein the syringe (12, 132) comprises a rolling diaphragm syringe (20), wherein the at least one interior surface comprises an inner surface of the syringe (12), and wherein the piston (103) is releasably connected to a proximal end wall of the syringe (12) and is configured to reciprocally move the proximal end wall of the syringe (12).

8. The fluid injector system (100) of claim 1, wherein driving the actuator to at least partially fill the at least one fluid reservoir comprises moving a piston (103) of the fluid injector system (100) operatively connected to the at least one fluid reservoir in a first direction, wherein driving the actuator to generate the at least partial vacuum within the internal volume comprises moving the piston (103) in the first direction, and wherein driving the actuator to expel the single coalesced bubble (62) comprises moving the piston (103) in a second direction opposite the first direction.

9. The fluid injector system (100) of claim 1 , wherein the at least one processor (904) is further programmed or configured to vibrate or oscillate at least one of the at least one interior surfaces to dislodge the one or more gas bubbles (61).

10. The fluid injector system (100) of claim 2 , wherein the at least one fluid reservoir further comprises a valve (136) in fluid communication with the outlet of the at least one fluid reservoir, wherein the valve (136) has at least a first open position and a second closed position, and wherein closing the outlet of the at least one fluid reservoir comprises moving the valve (136) to the second closed position.

11. The fluid injector system (100) of claim 10, wherein there is fluid communication between the internal volume of the at least one fluid reservoir and the fluid source (120) when the valve (136) is in the first open position, and wherein the valve (136) further comprises a third open position allowing fluid communication between the internal volume of the at least one fluid reservoir and a fluid outlet pathway (152).

12. The fluid injector system (100) of claim 1 , wherein the at least partial vacuum generated within the internal volume is sufficient to extract at least a portion of a dissolved gas from the fluid, and wherein the dissolved gas that is extracted coalesces into the single coalesced bubble (62).

13. The fluid injector system (100) of claim 1, wherein the at least one processor (904) is further programmed or configured to drive the actuator to prime a fluid path set (17) in fluid communication with a fluid outlet pathway (152) after the single coalesced bubble (62) is expelled from the at least one fluid reservoir.

14. The fluid injector system (100) of claim 1 , wherein the at least one processor (904) is further programmed or configured to:

measure the pressure within the internal volume of the fluid reservoir; and

adjust the at least partial vacuum within the internal volume based on the measured pressure.

15. The fluid injector system (100) of claim 14, wherein adjusting the at least partial vacuum comprises at least one of:

increasing or decreasing a speed of retraction of the actuator; and
increasing or reducing a diameter of at least a portion of a fluid path set (17) in fluid communication with the internal volume of the fluid reservoir.

**Patentansprüche**

1. Fluidinjektorsystem (100), Folgendes umfassend:

mindestens ein Fluidreservoir, das mindestens eine Innenfläche aufweist und ein Innenvolumen definiert;
mindestens einen Aktuator, der eingerichtet ist, um das Innenvolumen des mindestens einen Fluidreservoirs (132) zu verändern; und
mindestens einen Prozessor (904), der programmiert oder eingerichtet ist, um:

den Aktuator anzutreiben, um das mindestens eine Fluidreservoir mindestens teilweise mit einem Fluid aus einer Fluidquelle (120) zu füllen;
den Aktuator anzutreiben, um mindestens ein teilweises Vakuum in dem Innenvolumen zu erzeugen, um eine oder mehrere Gasblasen (61) zu lösen, die an der mindestens einen Innenfläche haften, und um zu bewirken, dass die eine oder die mehreren Gasblasen (61) sich zu einer einzigen koaleszierten Blase (62) vereinigen; und
den Aktuator anzutreiben, um die einzelne koaleszierte Blase (62) aus einem Auslass des mindestens einen Fluidreservoirs auszustoßen;
**dadurch gekennzeichnet, dass** der mindestens eine Prozessor (904) weiterhin programmiert oder eingerichtet ist, um den notwendigen Aktuatorversatz zu bestimmen und einzuleiten, der erforderlich ist, um das teilweise Vakuum zu erreichen, um die eine oder die mehreren Gasblasen (61) von der mindestens einen Innenfläche auf der Grundlage von mindestens einem von einer Oberflächenspannung des Fluids, einer Oberflächenspannung des Gases, einer Oberflächentextur der mindestens einen Innenfläche, einer Grenzflächenenergie zwischen der mindestens einen Innenfläche und dem Gas, einer Masse des Fluids über der einen oder den mehreren Gasblasen (61) in dem Fluidreservoir und einem Auftrieb der einen oder der mehreren Gasblasen (61) in dem Fluid zu lösen.

2. Fluidinjektorsystem (100) nach Anspruch 1, wobei der mindestens eine Prozessor (904) weiterhin programmiert oder eingerichtet ist, um vor dem Antreiben des Aktuators, um mindestens das teilweise Vakuum in dem Innenvolumen zu erzeugen, den Auslass des mindestens einen Fluidreservoirs zu schließen, um das Innenvolumen fluidisch zu isolieren.

3. Fluidinjektorsystem (100) nach Anspruch 2, wobei der mindestens eine Prozessor (904) weiterhin programmiert oder eingerichtet ist, um nach Schließen des Auslasses des mindestens einen Fluidreservoirs und nach Erzeugen des teilweisen Vakuums in dem Innenvolumen den Aktuator anzutreiben, um die einzelne koaleszierte Blase (62) unter Druck zu setzen.

4. Fluidinjektorsystem (100) nach Anspruch 3, wobei der mindestens eine Prozessor (904) weiterhin programmiert oder eingerichtet ist, um vor dem Antreiben des Aktuators, um die einzelne koaleszierte Blase (62) aus einem Auslass des mindestens einen Fluidreservoirs auszustoßen, den Auslass des mindestens einen Fluidreservoirs zu öffnen, um eine Fluidverbindung zwischen dem Innenvolumen und entweder der Fluidquelle (120) oder einem Fluidauslassweg (152) bereitzustellen.

5. Fluidinjektorsystem (100) nach Anspruch 1, wobei das mindestens eine Fluidreservoir eine Spritze (12, 132) umfasst und wobei der Aktuator einen Kolben (103) umfasst, der eingerichtet ist, um sich hin und her zu bewegen, um das Innenvolumen der Spritze (12, 132) zu verändern.

6. Fluidinjektorsystem (100) nach Anspruch 5, wobei die Spritze (12, 132) weiterhin einen Stößel (14) umfasst, der in der Spritze (12, 132) verschiebbar ist, wobei die mindestens eine Innenfläche weiterhin eine Oberfläche des Stößels (14) umfasst und wobei der Kolben (103) lösbar mit dem Stößel (14) verbunden ist und eingerichtet ist, um den Stößel (14) in der Spritze (12, 132) hin und her zu bewegen.

7. Fluidinjektorsystem (100) nach Anspruch 5, wobei die Spritze (12, 132) eine Rollmembranspritze (20) umfasst, wobei die mindestens eine Innenfläche eine Innenfläche der Spritze (12) umfasst und wobei der Kolben (103) lösbar mit einer proximalen Endwand der Spritze (12) verbunden ist und eingerichtet ist, um die proximale Endwand der Spritze (12) hin und her zu bewegen.

8. Fluidinjektorsystem (100) nach Anspruch 1, wobei Antreiben des Aktuators, um das mindestens eine Fluidreservoir mindestens teilweise zu füllen, Bewegen eines Kolbens (103) des Fluidinjektorsystems (100), das operativ mit dem mindestens einen Fluidreservoir verbunden ist, in eine erste Richtung umfasst, wobei Antreiben des Aktuators, um das mindestens teilweise Vakuum in dem Innenvolumen zu erzeugen, Bewegen des Kolbens (103) in die erste Richtung umfasst, und wobei Antreiben des Aktuators, um die einzelne koaleszierte Blase (62) auszustoßen, Bewegen des Kolbens (103) in eine zweite Richtung umfasst, die der ersten Richtung entgegengesetzt ist.

9. Fluidinjektorsystem (100) nach Anspruch 1, wobei der mindestens eine Prozessor (904) weiterhin programmiert oder eingerichtet ist, um mindestens eine der mindestens einen Innenfläche zu vibrieren oder zu oszillieren, um die eine oder die mehreren Gasblasen (61) zu lösen.

10. Fluidinjektorsystem (100) nach Anspruch 2, wobei das mindestens eine Fluidreservoir weiterhin ein Ventil (136) umfasst, das in Fluidverbindung mit dem Auslass des mindestens einen Fluidreservoirs steht, wobei das Ventil (136) mindestens eine erste offene Position und eine zweite geschlossene Position aufweist und wobei Schließen des Auslasses des mindestens einen Fluidreservoirs Bewegen des Ventils (136) auf die zweite geschlossene Position umfasst.

11. Fluidinjektorsystem (100) nach Anspruch 10, wobei es eine Fluidverbindung zwischen dem Innenvolumen des mindestens einen Fluidreservoirs und der Fluidquelle (120) gibt, wenn das Ventil (136) in der ersten offenen Position ist, und wobei das Ventil (136) weiterhin eine dritte offene Position umfasst, die eine Fluidverbindung zwischen dem Innenvolumen des mindestens einen Fluidreservoirs und einem Fluidauslassweg (152) ermöglicht.

12. Fluidinjektorsystem (100) nach Anspruch 1, wobei das mindestens teilweise Vakuum, das in dem Innenvolumen erzeugt wird, ausreichend ist, um mindestens einen Teil eines gelösten Gases aus dem Fluid auszutreiben, und wobei sich das gelöste Gas, das ausgetrieben wird, mit der einzelnen koaleszierten Blase (62) vereinigt.

13. Fluidinjektorsystem (100) nach Anspruch 1, wobei der mindestens eine Prozessor (904) weiterhin programmiert oder eingerichtet ist, um den Aktuator anzutreiben, um einen Fluidwegsatz (17) in Fluidverbindung mit einem Fluidauslassweg (152) vorzufüllen, nachdem die einzelne koaleszierte Blase (62) aus dem mindestens einen Fluidreservoir ausgestoßen wurde.

14. Fluidinjektorsystem (100) nach Anspruch 1, wobei der mindestens eine Prozessor (904) weiterhin programmiert oder eingerichtet ist, um:

den Druck innerhalb des Innenvolumens des Fluidreservoirs zu messen; und
das mindestens teilweise Vakuum innerhalb des Innenvolumens auf der Grundlage des gemessenen Drucks einzustellen.

15. Fluidinjektorsystem (100) nach Anspruch 14, wobei Einstellen des mindestens teilweisen Vakuums mindestens eines des Folgenden umfasst:

Erhöhen oder Verringern einer Rückzugsgeschwindigkeit des Aktuators; und
Erhöhen oder Reduzieren eines Durchmessers mindestens eines Abschnitts eines Fluidwegsatzes (17) in Fluidverbindung mit dem Innenvolumen des Fluidreservoirs.

## Revendications

1. Système d'injection de fluide (100), comprenant :

au moins un réservoir de fluide ayant au moins une surface intérieure et définissant un volume interne ;

au moins un actionneur configuré pour modifier le volume interne de l'au moins un réservoir de fluide (132) ; et
au moins un processeur (904) programmé ou configuré pour :

entraîner l'actionneur pour remplir au moins partiellement l'au moins un réservoir de fluide avec un fluide provenant d'une source de fluide (120) ;

entraîner l'actionneur pour créer au moins un vide partiel à l'intérieur du volume interne pour déloger une ou plusieurs bulles de gaz (61) adhérant à l'au moins une surface intérieure et pour faire coalescer la ou les bulles de gaz (61) en une unique bulle coalescée (62) ; et

entraîner l'actionneur pour expulser l'unique bulle coalescée (62) depuis une sortie de l'au moins un réservoir de fluide ;

**caractérisé en ce que** l'au moins un processeur (904) est en outre programmé ou configuré pour déterminer et initier le déplacement nécessaire de l'actionneur, nécessaire pour atteindre le vide partiel pour déloger la ou les bulles de gaz (61) de l'au moins une surface intérieure sur la base d'au moins un paramètre parmi une tension superficielle du fluide, une tension superficielle du gaz, une texture de surface de l'au moins une surface intérieure, une énergie de surface interfaciale entre l'au moins une surface intérieure et le gaz, un poids du fluide au-dessus de la ou des bulles de gaz (61) dans le réservoir de fluide, et une flottabilité de la ou des bulles de gaz (61) dans le fluide.

2. Système d'injection de fluide (100) selon la revendication 1, dans lequel l'au moins un processeur (904) est en outre programmé ou configuré pour, avant d'entraîner l'actionneur pour créer au moins le vide partiel à l'intérieur du volume interne, fermer la sortie de l'au moins un réservoir de fluide pour isoler fluidiquement le volume interne.

3. Système d'injection de fluide (100) selon la revendication 2, dans lequel l'au moins un processeur (904) est en outre programmé ou configuré pour, après la fermeture de la sortie de l'au moins un réservoir de fluide et après la création du vide partiel à l'intérieur du volume interne, entraîner l'actionneur pour pressuriser l'unique bulle coalescée (62).

4. Système d'injection de fluide (100) selon la revendication 3, dans lequel l'au moins un processeur (904) est en outre programmé ou configuré pour, avant d'entraîner l'actionneur pour expulser l'unique bulle coalescée (62) depuis une sortie de l'au moins un réservoir de fluide, ouvrir la sortie de l'au moins un réservoir de fluide pour assurer une communication fluidique entre le volume interne et soit la source de fluide (120), soit une voie de sortie de fluide (152).

5. Système d'injection de fluide (100) selon la revendication 1, dans lequel l'au moins un réservoir de fluide comprend une seringue (12, 132), et dans lequel l'actionneur comprend un piston (103) configuré pour effectuer un mouvement de va-et-vient pour modifier le volume interne de la seringue (12, 132).

6. Système d'injection de fluide (100) selon la revendication 5, dans lequel la seringue (12, 132) comprend en outre un piston plongeur (14) que l'on peut faire glisser à l'intérieur de la seringue (12, 132), dans lequel l'au moins une surface intérieure comprend en outre une surface du piston plongeur (14), et dans lequel le piston (103) est relié de façon détachable au piston plongeur (14) et est configuré pour imprimer un mouvement de va-et-vient au piston plongeur (14) à l'intérieur de la seringue (12, 132).

7. Système d'injection de fluide (100) selon la revendication 5, dans lequel la seringue (12, 132) comprend une seringue à membrane déroulante (20), dans lequel l'au moins une surface intérieure comprend une surface interne de la seringue (12), et dans lequel le piston (103) est relié de façon détachable à une paroi d'extrémité proximale de la seringue (12) et est configuré pour imprimer un mouvement de va-et-vient à la paroi d'extrémité proximale de la seringue (12).

8. Système d'injection de fluide (100) selon la revendication 1, dans lequel l'entraînement de l'actionneur pour remplir au moins partiellement l'au moins un réservoir de fluide comprend le déplacement d'un piston (103) du système d'injection de fluide (100) fonctionnellement relié à l'au moins un réservoir de fluide dans une première direction, dans lequel l'entraînement de l'actionneur pour créer le vide au moins partiel à l'intérieur du volume interne comprend le déplacement du piston (103) dans la première direction, et dans lequel l'entraînement de l'actionneur pour expulser l'unique bulle coalescée (62) comprend le déplacement du piston (103) dans une deuxième direction opposée à la première direction.

9. Système d'injection de fluide (100) selon la revendication 1, dans lequel l'au moins un processeur (904) est en outre programmé ou configuré pour faire vibrer ou osciller la surface intérieure ou au moins une des surfaces intérieures pour déloger la ou les bulles de gaz (61).

**10.** Système d'injection de fluide (100) selon la revendication 2, dans lequel l'au moins un réservoir de fluide comprend en outre une vanne (136) en communication fluidique avec la sortie de l'au moins un réservoir de fluide, dans lequel la vanne (136) a au moins une première position ouverte et une deuxième position fermée, et dans lequel la fermeture de la sortie de l'au moins un réservoir de fluide comprend le déplacement de la vanne (136) jusqu'à la deuxième position fermée.

**11.** Système d'injection de fluide (100) selon la revendication 10, dans lequel une communication fluidique existe entre le volume interne de l'au moins un réservoir de fluide et la source de fluide (120) quand la vanne (136) est dans la première position ouverte, et dans lequel la vanne (136) comprend en outre une troisième position ouverte permettant une communication fluidique entre le volume interne de l'au moins un réservoir de fluide et une voie de sortie de fluide (152).

**12.** Système d'injection de fluide (100) selon la revendication 1, dans lequel le vide au moins partiel créé à l'intérieur du volume interne est suffisant pour extraire au moins une partie d'un gaz dissous du fluide, et dans lequel le gaz dissous qui est extrait coalesce en l'unique bulle coalescée (62).

**13.** Système d'injection de fluide (100) selon la revendication 1, dans lequel l'au moins un processeur (904) est en outre programmé ou configuré pour entraîner l'actionneur pour amorcer une tubulure de fluide (17) en communication fluidique avec une voie de sortie de fluide (152) après que l'unique bulle coalescée (62) a été expulsée de l'au moins un réservoir de fluide.

**14.** Système d'injection de fluide (100) selon la revendication 1, dans lequel l'au moins un processeur (904) est en outre programmé ou configuré pour :

mesurer la pression à l'intérieur du volume interne du réservoir de fluide ; et
ajuster le vide au moins partiel à l'intérieur du volume interne sur la base de la pression mesurée.

**15.** Système d'injection de fluide (100) selon la revendication 14, dans lequel l'ajustement du vide au moins partiel comprend au moins une des opérations suivantes :

augmentation ou diminution d'une vitesse de rétraction de l'actionneur ; et
augmentation ou diminution d'un diamètre d'au moins une partie d'une tubulure de fluide (17) en communication fluidique avec le volume interne du réservoir de fluide.

FIG. 1

FIG. 2

FIG. 3A

FIG. 3B

Electronic control
device 900

Monitor

Speaker

930

934

Printer

Output
interface

936

932

System
memory
908

System
bus
906

Comm.
inter.

Comm.
device

Network
environment

Processing
unit
904

928

942

940

User input
interface

938

944

Computer

Hard disk
drive
912

Non
removable
memory
interface
910

Removable
memory
interface
914

923

Keyboard

924

Memory
card

926

Mouse

Computer
system
environment
902

916

920

Disk
drive
unit

Optical
disk
drive
unit

918

922

Disk

Compact
disk

USB port

921

FIG. 4

FIG. 5

FIG. 6A

FIG. 6B

800

| Draw initial volume of fluid into fluid reservoir | 802 |
|---|---|

↓

| Transform fluid injector system to closed system | 804 |
|---|---|

↓

| Place at least partial vacuum on fluid reservoir | 806 |
|---|---|

↓

| Apply at least one force to fluid injector sytem | 808 |
|---|---|

↓

| Pressurize fluid reservoir (optional) | 810 |
|---|---|

↓

| Transform fluid injector system to open system | 812 |
|---|---|

↓

| Purge coalesced gas bubble | 814 |
|---|---|

↓

| Fill fluid reservoir to total volume | 816 |
|---|---|

FIG. 7

FIG. 8A

FIG. 8B

FIG. 8C

FIG. 9

FIG. 10A

FIG. 10B

850

852 — Draw initial volume of fluid into fluid reservoir

854 — Place at least partial vacuum on fluid reservoir by increasing piston speed

866 — Place at least partial vacuum on fluid reservoir by reducing diameter of fluid path

856 — Measure pressure in fluid reservoir

868 — Measure pressure in fluid reservoir

858 — Does measured pressure meet desired pressure drop?

No

870 — Does measured pressure meet desired pressure drop?

No

860 — Fill reservoir to total volume

Yes

872 — Fill reservoir to total volume

Yes

862 — Purge coalesced gas bubble

874 — Purge coalesced gas bubble

864 — Refill fluid reservoir to total volume

876 — Return diameter of fluid path set to original diameter and refill fluid reservoir to total volume

FIG. 11

**FIG. 12A**  **FIG. 12B**

FIG. 13A

FIG. 13B

FIG. 14A

FIG. 14B

EP 3 781 234 B1

FIG. 15

FIG. 16

FIG. 17

**EP 3 781 234 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100331779 A1
- WO 2017096072 A1
- WO 2007133942 A2
- US 7553294 B
- US 7563249 B
- US 8945051 B
- US 9173995 B
- US 10124110 B
- US 305285
- US 15541573
- US 15568505
- US 2016012434 W
- US 20160331951 A
- US 20170035974
- US 20180261496
- WO 2018075379 A
- WO 2018075386 A